# EUROPEAN PATENT APPLICATION

(11) **EP 4 710 948 A1**
(43) Date of publication of application: **18.03.2026**
(21) Application number: 24306514.1
(22) Date of filing: 13.09.2024
(51) Int. Cl.: A61K 49/00, A61P 39/00, A61P 31/14

(54) **A RECOMBINANT SARS-COV-2 S GLYCOPROTEIN TRIMER, PROTEOLIPOSOMES COMPRISING SUCH A TRIMER AND THEIR USE AS A VACCINE**

(71) Applicant: Université Grenoble Alpes, 38400 Saint-Martin-d'Hères (FR); Commissariat à l'Energie Atomique et aux Energies Alternatives, 75015 Paris (FR); Centre Hospitalier Régional de Grenoble, 38043 Grenoble Cedex 9 (FR); Centre National de la Recherche Scientifique, 75016 Paris (FR)
(72) Inventor: WEISSENHORN, Winfried, 38180 SEYSSINS (FR); SULBARAN, Guidenn, 69160 TASSIN LA DEMI LUNE (FR); GUILLIGAY, Delphine, 38340 VOREPPE (FR); EFFANTIN, Gregory, 38180 SEYSSINS (FR); AMEN, Axelle, 38000 GRENOBLE (FR); POIGNARD, Pascal, 38000 GRENOBLE (FR); LE GRAND, Roger, 92290 CHATENAY-MALABRY (FR); LETSCHER, Hélène, 92140 CLAMART (FR)
(74) Representative: Germain Maureau

(57) **Abstract**

The present invention concerns a recombinant SARS-CoV-2 S glycoprotein trimer stabilized in a conformation that is anterior to the post-fusion conformation, as well as a proteoliposome comprising such a recombinant trimer and a vaccine based on such a proteoliposome. The invention also relates to a method of treating or preventing a SARS-CoV-2 infection in a subject using such a vaccine.

## Description

### FIELD OF THE INVENTION

The invention relates to the field of preventing and/or treating a coronavirus infection, in particular a SARS-CoV-2 infection. More particularly, the invention relates to a recombinant SARS-CoV-2 S glycoprotein trimer stabilized in a conformation that is anterior to the post-fusion conformation, as well as a proteoliposome comprising such a recombinant trimer and a vaccine based on such a proteoliposome. The invention also relates to a method of treating or preventing a SARS-CoV-2 infection in a human being using such a vaccine.

### STATE OF THE ART

The severe acute respiratory syndrome coronavirus 2, SARS-CoV-2, a beta-coronavirus, is the etiological agent of coronavirus disease 2019 (COVID-19), which emerged at the end of 2019, profoundly disrupting the entire world. This unprecedented health crisis has posed significant challenges, testing the resilience of societies, healthcare systems, and the global economy. The initial cases were reported in the city of Wuhan, China, before rapidly spreading worldwide, prompting governments across the globe to implement drastic measures to contain the virus's spread. Such a pandemic caused more than 5 million deaths as of November 2021 and has highlighted the urgent need for effective infection control and prevention.

An important correlate of protection of antiviral vaccines is the generation of neutralizing antibodies (nAb). The main SARS-CoV-2 target for inducing neutralizing antibodies is the spike (S) glycoprotein, which plays an essential role in virus attachment, fusion and entry into host cells, in particular due to its surface location. The S glycoprotein is composed of the S1 subunit that harbors the receptor-binding domain (RBD) and the S2 membrane fusion subunit that anchors the S trimer in the virus membrane.

RBD binding to the cellular receptor Angiotensin-converting enzyme 2 (ACE2) leads to virus attachment and subsequent S2-mediated fusion with endosomal membranes establishes infection. The S glycoprotein is synthesized as a trimeric precursor polyprotein that is proteolytically cleaved by furin and furin-like proteases in the Golgi generating the non-covalently linked S1-S2 heterotrimer.

The structure of the S glycoprotein reveals a compact heterotrimer composed of S1 (NTD, RBD, RBM and two subdomains), S2 (the transmembrane region) and a cytoplasmic domain. The conformation of RBD is in a dynamic equilibrium between either all RBDs in a closed, receptor-inaccessible conformation or one or two RBDs in the "up", receptor-accessible, conformation. Only S RBD in the 'up' position allows receptor binding, which leads to endocytosis of the virion and to triggering the S2 post fusion conformation in proteolytically cleaved S glycoprotein {1; 2}.

Current licensed vaccines and vaccines tested in preclinical settings focus on either the complete S glycoprotein or RBD {3}. Notably, RBD is highly immunodominant and targeted by approximately 90% of the neutralizing antibody response present in SARS-CoV-2 immune sera post infection {4; 5; 6} or generated upon vaccination {7}.

The magnitude of antibody responses to S glycoprotein during natural infection varies greatly and correlates with disease severity and duration. Basal responses are generally maintained for months or decline within weeks after infection, notably in asymptomatic individuals. Thus, any vaccine-based approach aims to induce long-lasting immunity.

A number of animal models have been developed to study SARS CoV-2 infection including the macaque model, which demonstrated induction of innate, cellular and humoral responses upon infection conferring partial protection against reinfection {8}. Consequently, many early vaccine candidates provided protection in the macaque model including the currently licensed vaccines based on S-specific mRNA delivery (BNT162b2, Pfizer/BioNTech; mRNA-1273, Moderna), adenovirus vectors (ChAdOx1 nCoV-19, Oxford/AstraZeneca; Ad26.COV2.S, Johnson & Johnson) and inactivated SARS-CoV-2 (PiCoVacc/CoronaVac, Sinovac). Employing the classical subunit approach, S glycoprotein subunit vaccine candidates have generated different levels of neutralizing antibody responses in preclinical testing {9}.

However, continuous SARS-CoV-2 mutations have led to the emergence of multiple variants of concern (VOCs), including Alpha, Beta, Gamma, Delta, and Omicron {10}. Notably, Omicron has further mutated into many subvariants including BA2 and XBB variants, which rendered them prone to immune evasion provided by current vaccines and therapeutic antibodies targeting the original strain or previous VOCs {11; 12; 13; 14; 15}. These subvariants carry more than 30 mutations within their spike, most of them within RBD that contributes to higher transmission rates {16} and neutralizing antibody escape {17; 18; 19; 20}. Current subvariants XBB and BQ1.1 are most potent in escaping neutralizing antibody (nAbs) generated by Wuhan strain S-based vaccines {21; 20}. As a consequence, the monovalent SARS CoV-2 vaccines are not effective in preventing infection {22; 23; 24; 18} nor transmission {25}, but still provide protection against severe disease {26; 27}. In other words, the vaccine- or earlier virus infection-acquired antibody repertoire proved to be insufficient to fully protect against later VOCs while remaining partially efficient to protect against severe covid-19 disease {28}.

Although the immune response can be boosted with mRNA vaccines {29; 30}, second generation bivalent vaccines have been developed that increase neutralizing antibody (nAb) titers showing cross-reactivity against Omicron {31; 32}. Nevertheless, the generation of an effective potent immune response by bivalent booster vaccines has been challenged {33}.

At present, there is no vaccine against SARS-CoV-2 that can ensure a strong immune response as well as an effective immune response against the majority of future variants resulting from new mutations in the virus.

Therefore, there is a genuine need to develop a functional vaccine that would ensure a robust immune response against future mutants of the virus, thereby ensuring strong vaccination coverage of the population against these future mutants.

### DESCRIPTION OF THE INVENTION

An objective of the present invention is to provide a recombinant SARS-CoV-2 S glycoprotein trimer that allows preventing and/or treating a coronavirus infection, in particular a SARS-CoV-2 infection.

Thus, another objective of the present invention is to provide a vaccine based on such a recombinant SARS-CoV-2 S glycoprotein trimer.

In order to circumvent the high mutation rate within RBD and focus the immune response away from RBD towards other conserved epitopes of S glycoprotein targeted by broadly neutralizing antibodies such as the fusion peptide, the stem helix or SD1 {34}, the inventors have now discovered that a recombinant SARS-CoV-2 S glycoprotein trimer comprising specific modifications, efficiently protects animals to which it is administered from infections by at least wild-type SARS-CoV-2 and Alpha pseudovirus variants, and neutralizes Beta and Gamma pseudovirus variants at reduced potency while at the same time being highly immunogenic against the majority of future variants resulting from new mutations in the virus. Indeed, the inventors have developed a recombinant SARS-CoV-2 S glycoprotein trimer, stabilized in a conformation that is anterior to the post-fusion conformation, which has RBD deleted. The inventors have demonstrated that such a recombinant is particularly effective when integrated into in synthetic virus-like particles employing liposomes. Consequently, the invention enables more robust vaccination coverage against future mutants within a population that has already received double vaccination with currently available vaccines.

Accordingly, the present invention relates to a recombinant SARS-CoV-2 S glycoprotein trimer comprising three protomers each comprising a subunit S1 (S1) which may be identical or different in each protomer and a subunit S2 (S2) which may be identical or different in each protomer, characterised in that at least one of the protomer is a recombinant protomer comprising a subunit S1 devoid of receptor-binding domain (RBD),
wherein said recombinant SARS-CoV-2 S glycoprotein trimer has a conformation that is anterior to the post-fusion conformation.

According to the invention, the immune response is directed away from the receptor-binding domain (RBD), which constitutes 44% of the mutations (15 in total) observed in the original Omicron variant B.1.1.529. Instead, it is redirected towards other sites on the S protein that have shown lesser susceptibility to mutations since the emergence of the initial strain of the virus.

In the context of the invention, the term "recombinant SARS-CoV-2 S glycoprotein trimer" refers to a protein synthesized in the laboratory that mimics the spike (S) protein of the SARS-CoV-2 virus, the causative agent of COVID-19. This spike protein (also called SARS-CoV-2 S glycoprotein trimer) is crucial for viral entry into host cells. Through genetic recombination techniques, the viral genetic material is inserted into host cells, such as yeast or mammalian cells, which then express the viral spike protein. This trimeric recombinant protein comprises three subunits known as protomers, each representing a monomer of the spike protein.

In the context of the invention, the term "protomer" means a fundamental structural unit that composes a protein or a macromolecule. In other words, it is the smallest unit that makes up the oligomeric form of a protein or a macromolecular complex. Protomers can bind together to form larger and more complex structures, such as homo-oligomers or multimeric proteins.

In the context of the invention, the term "proteoliposome" means a nanostructure forming a lipid vesicle composed of a lipid bilayer, similar to the cell membrane or a virus membrane, within which membrane proteins are incorporated. These proteins can be integrated into the lipid bilayer or attached to its external surface via non-covalent bonds, such as affinity tags or covalent bonds via SH groups of cysteine residues or other chemical compositions. Proteins, such as viral glycoproteins are generally attached via their C-terminal end, but membrane attachment tags or residues can be incorporated at suitable places of the glycoprotein to change the attachment orientation.

In the context of the invention, the expression "a conformation that is anterior to the post-fusion conformation" refers to the structural state of the trimeric protein complex before it undergoes the conformational changes associated with membrane fusion. This includes the pre-fusion conformation, which is the initial, stable form of the protein complex, as well as any intermediate conformations that occur during the transition from the pre-fusion to the post-fusion state. The anterior conformation is characterized by specific structural features and arrangements of the protein subunits that differ from those observed in the post-fusion state. These conformations are critical for the initial stages of the viral entry process.

In the context of this invention, the term 'the pre-fusion conformation' refers to the structural state of the spike (S) protein before it undergoes the conformational changes associated with membrane fusion. This pre-fusion conformation represents the initial, stable form of the S protein, characterized by specific structural features and arrangements of the protein subunits that are distinct from those observed in the post-fusion state and from any intermediate conformations that occur during the transition.

In the context of this invention, the term 'non-antigenic linker' refers to a molecular bridge designed to connect two biological entities without eliciting an immune response. This linker is specifically engineered to be biocompatible and to minimize recognition by the immune system, thereby preventing the activation of immune responses that could lead to adverse effects.

In the context of the invention, the expression "any sequence sharing an identity of at least 80% with a sequence (nucleic or amino acid sequence)" corresponds to any sequence sharing an identity of at least 80%, or 81%, 82%, 83%, 84%, 85%, 86%, 87%, 88%, 89%, 90%, 91%, 92%, 93%, 94%, 95% or even 96%, 97%, 98%, or 99% with said sequence.

In the context of this invention, the expression "at least one epitope is linked to a glycan" refers to the covalent or non-covalent attachment of at least one specific epitope, which is a distinct part of an antigen that is recognized by the immune system, to a glycan, which is a carbohydrate or carbohydrate chain that may consist of multiple sugar molecules bonded together. This linkage can occur through various biochemical mechanisms, including but not limited to glycosylation processes, where the glycan is either directly or indirectly bound to the epitope. Such configurations can be important for the functionality and efficacy of the trimer of the invention, as they can influence the immunogenic properties and stability of the resulting glycoprotein or conjugate. This association may be significant for the intended application of the invention, by facilitating the recognition and response of the immune system towards another specific part of the trimer of the invention (namely: its S2 subunit).

In the context of this invention, the term "at least one epitope is unlinked to a glycan that initially hid said epitope" refers to the condition where at least one specific epitope, which is a distinct part of an antigen recognized by the immune system, has been separated from a glycan that was previously attached to it and obscured the epitope from immune recognition. This detachment ensures that the epitope is no longer masked by the glycan, thereby restoring its accessibility and immunogenic properties. This process can be important for the functionality and efficacy of the trimer of the invention, as it allows the epitope to be exposed and identified by the immune system or other detection mechanisms, facilitating enhanced recognition and response by the immune system of the specific part of the trimer of the invention comprising said hid epitope(s) (namely: its S2 subunit).

Preferably, the subject of the present invention is a recombinant SARS-CoV-2 S glycoprotein trimer as defined above having the following technical features, taken alone or in combination:
- said recombinant protomer comprises a non-antigenic linker linking N-terminal domain (NTD) and subunit S2, said non-antigenic linker preferably being an amino acid motif of sequence consisting of at least three amino acids, more preferably the amino acid motif of sequence consisting of amino acid sequence SEQ ID NO: 12;
- said recombinant protomer comprises a C-terminal domain (CTD) and a terminal linker linking CTD end, said terminal linker being a poly-histidine tag selected from the group consisting of:
   - non-covalent linker with linkage His-tag interaction with DGS-NTA lipids, such as one of the amino acid sequences chosen among amino acid sequences SEQ ID NO: 13, SEQ ID NO: 14 and SEQ ID NO: 15,
   - covalent linker having an exposed C-terminal cysteine residue for a covalent linkage via with lipids containing a SH group, such as a linker consisting of the amino acid sequence SEQ ID NO: 5, lipids containing a bromoacetic acid cholesterol or lipids containing a cholesterol-PEG12-maleimide,
   - linker with cholesterol modification of the C-terminus cysteine or any other exposed cysteine residue for spontaneous membrane anchoring;
- said recombinant protomer comprises a cleavage site situated between subunit S1 and subunit S2, said cleavage site being preferably a furin or furin-like cleavage site.

In order to better stabilizing said recombinant SARS-CoV-2 S glycoprotein trimer in its conformation, the subject of the present invention is a recombinant SARS-CoV-2 S glycoprotein trimer as defined above having the following technical features, taken alone or in combination:
- the subunits S2 of at least two of the three protomers are cross-linked to each other, preferably the subunits S2 of the three protomers being cross-linked to each other or
- said subunits S2 comprise each at least one engineered cystein mutant forming inter-protomers disulfide bonds, preferably said at least one engineered cystein being formed by at least one of the further engineered modifications made in the amino acid sequence of SEQ ID NO: 1:
   - asparagine residue at position 786 is replaced by cysteine residue and threonine residue at position 355 is replaced by cysteine residue,
   - asparagine residue at position 777 is replaced by cysteine residue and glutamine residue at position 563 is replaced by cysteine residue.

In order to facilitate enhanced recognition and response by the immune system of the towards the S2 subunit, the subject of the present invention is a recombinant SARS-CoV-2 S glycoprotein trimer as defined above having the following technical features, taken alone or in combination:
- at least one epitope of N-terminal domain (NTD) of said recombinant protomer is linked to a glycan, preferably at least one of the further engineered modifications is made in the amino acid sequence of SEQ ID NO: 1:
   - phenylalanine residue at position 135 is replaced by asparagine residue and asparagine 137 is replaced by serine,
   - arginine residue at position 158 is replaced by asparagine residue and tyrosine residue at position 160 is replaced by threonine residue,
   - glycine residue at position 89 is replaced by serine residue,
   - lysine residue at position 187 is replaced by serine residue,
   - arginine residue at position 190 is replaced by serine residue,
   - valine residue at position 213 is replaced by serine residue,
   - arginine residue at position 791 is replaced by asparagine residue,
   - aspartic acid residue at position 793 is replaced by serine residue or
- at least one epitope of subunit S2 is unliked to the glycan that initially hid said epitope, preferably at least one arginine residue chosen among arginine residues: N882, N906, N942, N966, N981 and N1002 of the amino acid sequence of SEQ ID NO: 1, is substituted by a glutamine residue.

Advantageously, the subject of the present invention is a recombinant SARS-CoV-2 S glycoprotein trimer as defined above having the following technical features, taken alone or in combination:
- said trimer has a pre-fusion conformation;
- said recombinant protomer has an amino acid sequence selected from the group consisting of the sequences having at least 80% amino acid identity with the amino acid sequence of SEQ ID NO: 1;
- the three protomers are devoid of receptor-binding domain (RBD), the three protomers having an amino acid sequence selected from the group consisting of the sequences having at least 80% amino acid identity with the amino acid sequence of SEQ ID NO: 1;
- said trimer is a homomeric trimer;
- said trimer is formulated as mRNA, preferably, said recombinant SARS-CoV-2 S glycoprotein trimer being formulated as mRNA-lipid nanoparticles (LNP).

The invention also relates to a method of producing a recombinant SARS-CoV-2 S glycoprotein trimer as defined above, comprising the following steps:
- expressing nucleic acid molecule(s) encoding said recombinant protomer(s) in a host cell to produce said recombinant SARS-CoV-2 S glycoprotein trimer, and
- purifying said recombinant SARS-CoV-2 S glycoprotein trimer.

Advantageously, said method of producing a recombinant SARS-CoV-2 S glycoprotein trimer further comprises a step of treating said recombinant SARS-CoV-2 S glycoprotein trimer with formaldehyde according to claim.

Furthermore, the invention also relates to a proteoliposome comprising a lipid vesicle a surface of which is coated by the recombinant SARS-CoV-2 S glycoprotein trimer according to one of the variants of the invention. Advantageously, said lipid vesicle comprises 50% to 70% by weight of L-α-phosphatidylcholine, 30% to 45% by weight of cholesterol and 0.1% to 10% by weight of a polyhistidine-tag conjugating lipid, preferably 60% by weight of L-α-phosphatidylcholine, 36% by weight of cholesterol and 4% by weight of a polyhistidine-tag conjugating lipid.

The invention also relates to a method of preparing a proteoliposome as defined above comprising the step of incubating a recombinant SARS-CoV-2 S glycoprotein trimer according to any of claims 1 to 13 with a lipid vesicle.

In particular embodiments, a method of preparing a proteoliposome according to the invention comprises incubating the recombinant SARS-CoV-2 S glycoprotein trimer of the invention with the lipid vesicle, so as to ensure immobilization of the trimer on the surface of the lipid vesicle by non-covalent interactions. This incubation is preferably carried out for at least 6 hours, preferably from 12 to 24 hours, and preferably at a temperature of between 20 and 25 °C. The ratio glycoprotein/lipid vesicle is preferably chosen so as to have a sufficient excess of protein to saturate the lipid vesicles, more particularly at least 2 times of excess protein. The ratio glycoprotein/lipid vesicle is for example 3:1 w:w. (range 2:1 minimum or 4:1 maximum). Alternatively, the recombinant trimer of the invention can be covalently linked to the surface of the lipid vesicle, for example by reacting at least a cysteine residue of at least a protomer of the trimer, preferably a cysteine residue situated in a C-terminal part of the protomer, with a group carried by the lipid vesicle, capable of reacting with a sulfhydryl group.

Furthermore, the invention also relates to a vaccine comprising a recombinant SARS-CoV-2 S glycoprotein trimer according to one of the variants of the invention or a proteoliposome as defined above and a pharmaceutically acceptable carrier and/or adjuvant. Advantageously, said vaccine is in a unit dose comprising 10 to 100 µg of said proteoliposome. Advantageously, said vaccine is for use for the preventive or curative treatment of a coronavirus infection, in particular a SARS-CoV-2 infection.

The invention also relates to a method for the preventive or curative treatment of a coronavirus infection in a human being, in particular a SARS-CoV-2 infection in a human being, by administering a vaccine as defined above. Advantageously, said vaccine is administrated to the subject intramuscularly, oraly or mucosally such as intranasally.

The present invention is illustrated without limitation by the following examples.

### BRIEF DESCRIPTION OF THE FIGURES

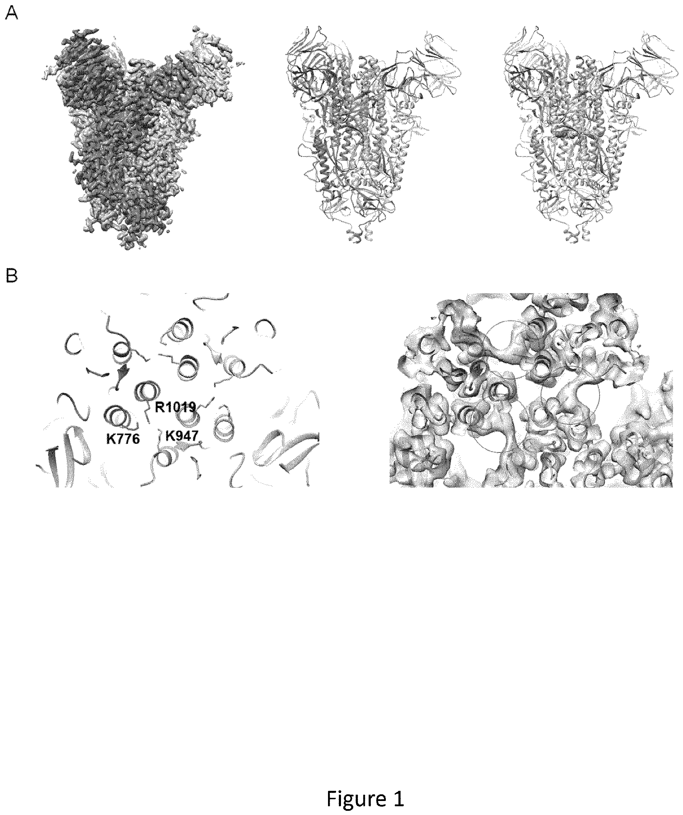

Figure 1 shows the results of structural characterization of a stabilized recombinant SARS-CoV-2 S glycoprotein trimer of the invention (SARS-CoV2 SΔRBD) and proteoliposomes based on this trimer coated onto lipid vesicles surface (SΔRBD-LV). (A) Left panel, cryo-EM coulomb potential map of SΔRBD; each protomer is colored differently. Middle panel, molecular model of SΔRBD shown as ribbon. Right panel, one major cross-linking site was identified that covalently link the S2 subunits from different protomers. (B) Close-up of the cross-linking sites between S2 subunits (left panel). Continuous density between the central helix R827 as well as S2 K584 to S2 HR1K755 suggested two alternative cross-links between protomers with equal occupancy (right panel).

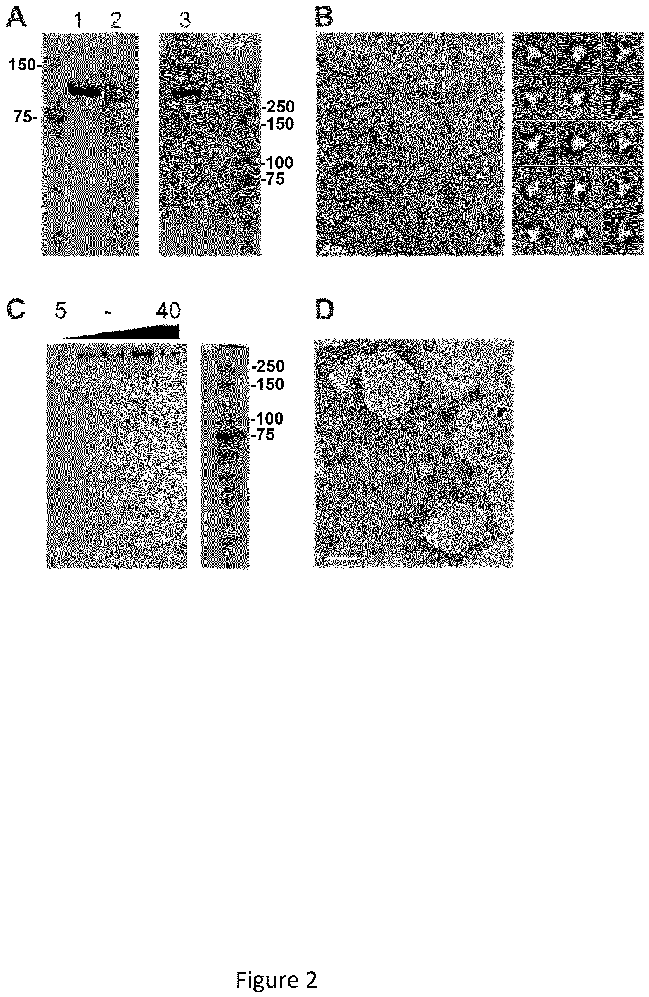

Figure 2 shows the expression and characterization of the SARS Cov-2 S glycoprotein trimer of the invention (SARS-CoV2 SΔRBD) and proteoliposomes based on this trimer coated onto lipid vesicles surface (SΔRBD-LV). (A) SDS-PAGE of purified S '6P' (lane 1), SΔRBD (lane 2) and FA cross-linked SΔRBD (lane 3). Molecular weight markers are indicated. (B) Negative staining electron microscopy of the SΔRBD (left panel) and 2-D class averages of the most populated classes (right panel) (Scale bar, 100 nm). (C) SDS-PAGE analysis of the SΔRBD-LVs purified by sucrose gradient density centrifugation. (D) FA-cross-linked S glycoprotein was incubated with liposomes containing 4% DGS-NTA lipids, purified by sucrose gradient density centrifugation and SDS-PAGE analysis as well as negative staining electron microscopy (D) showing decoration of the liposomes with SΔRBD (scale bar, 100 nm).

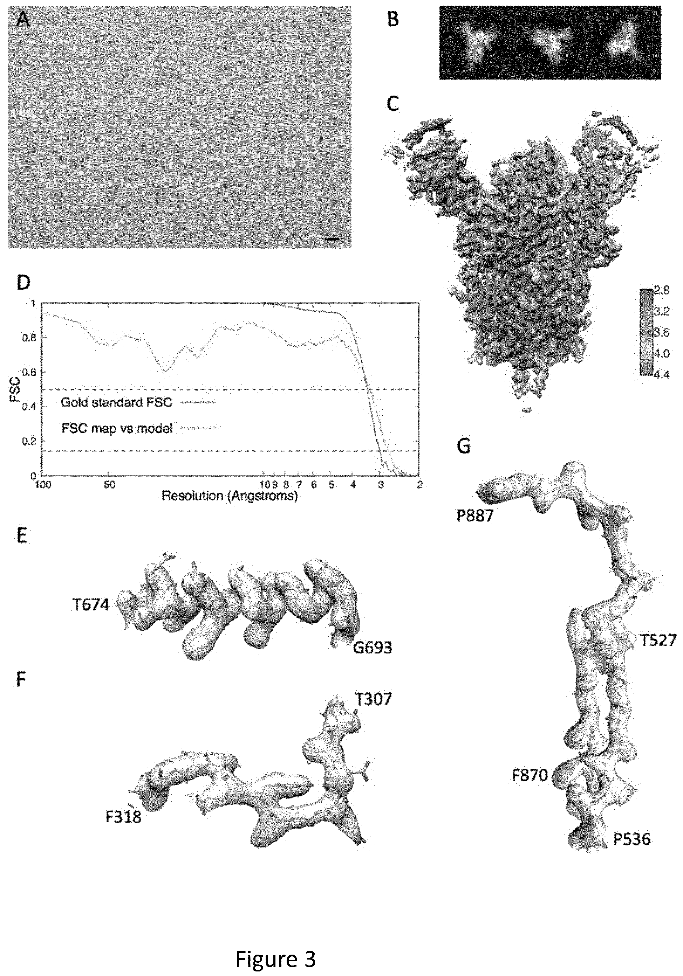

Figure 3 shows the Cryo-EM structure validation of the SARS Cov-2 S glycoprotein trimer of the invention (SARS-CoV2 SΔRBD) and proteoliposomes based on this trimer coated onto lipid vesicles surface (SΔRBD-LV). (A) Cryo-EM image of the SΔRBD trimer and (B) 2-D class average representatives. (C) Isosurface representation of the 3D reconstruction of the SΔRBD trimer obtained by cryo-EM colored by local resolution (color code in Å). (D) Fourier Shell Correlation (FSC) curves calculated between two independent 3D reconstructions of the SΔRBD trimer (gold standard FSC, resolution of 3.0Å at FSC=0.143) and between the cryo-EM 3D reconstruction and the refined atomic model (FSC map vs model, resolution of 3.3 Å at FSC=0.5). (E, F, G) Three different zones of the SΔRBD trimer are shown to illustrate both the high resolution of the 3D reconstruction (grey isosurface) and the quality of the refined atomic model. Some residue numbers are indicated for orientation.

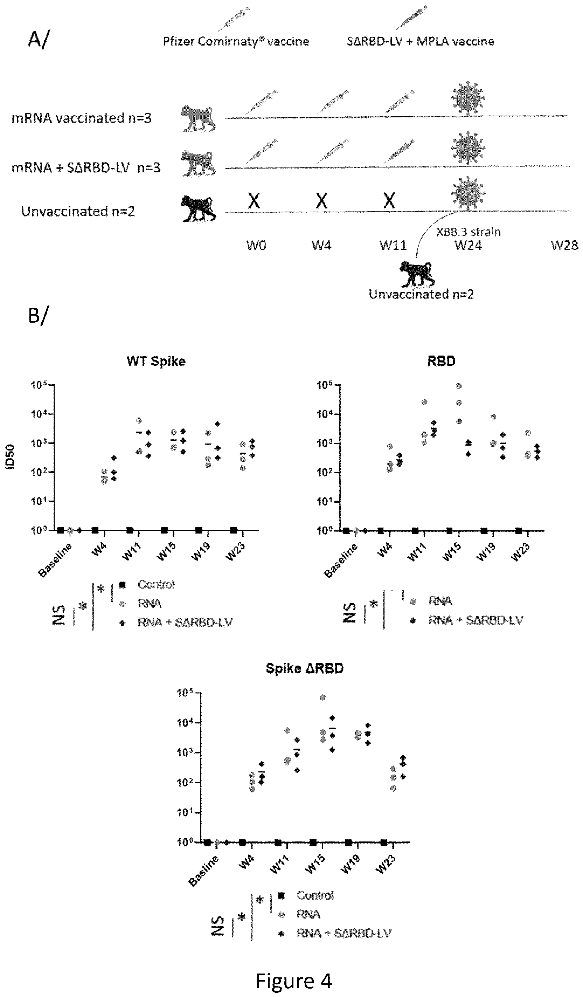

Figure 4 shows the results of analysis of antibody responses induced by SΔRBD-LV vaccination of cynomolgus macaques. (A) Scheme of vaccination, challenge and sampling. Syringes indicate the time points of vaccination, with the green syringe representing the Pfizer vaccine and the blue one the SΔRBD-LV. The virus particle represents the time point of challenge. Symbols of identifying individual macaques are used in all figures. (B) ELISA of SARS-CoV-2 S-protein-specific IgG determined during the study at weeks 0, 4, 11 and 15. Mean Ab titers of two experiments of individual animals are shown. Differences between matched groups were compared using the Kruskal-Wallis and Mann-Whitney non parametric rank test.

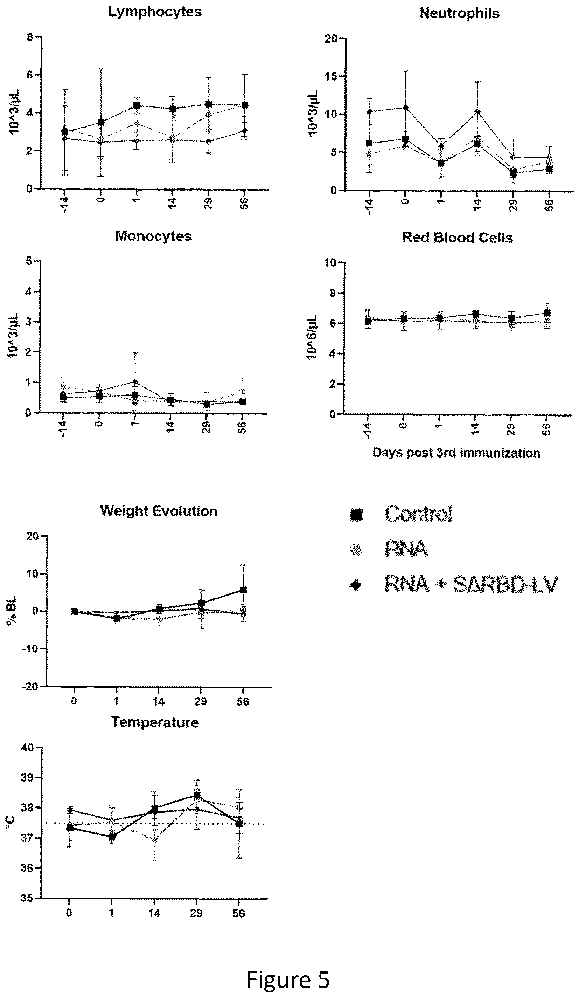

Figure 5 shows the safety profile of the SΔRBD-LV vaccine. Several clinical parameters were recorded after the differential immunization. Circulating lymphocytes, monocytes, neutrophils and Red blood cells were counted on EDTA-K3 tubes. Weight evolution was calculated as a % evolution of the considered baseline, here 14 days before the third immunization. Rectal temperature was obtained on anesthetized animals.

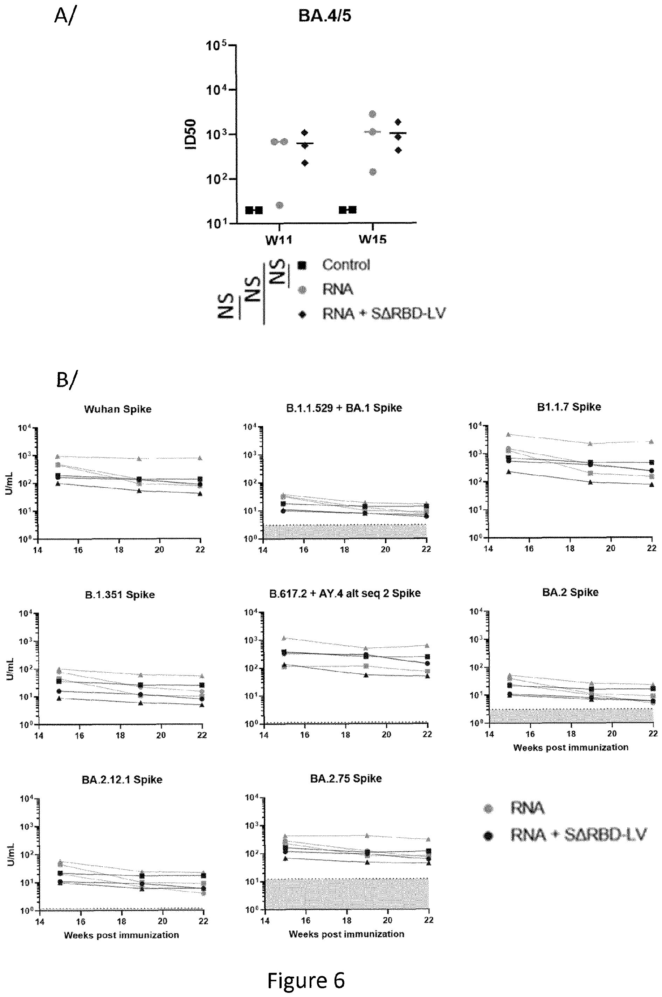

Figure 6 shows the serum neutralization of SARS-CoV-2 pseudovirus upon SΔRBD-LV vaccination. (A) Various SARS-CoV-2 strain virus neutralization are shown for sera collected at weeks 11 and 15. Bars indicate mean titers of the three animals. Neutralization is expressed as inhibitory dilution at which 50% neutralization is achieved. Differences between groups were compared using the Kruskal-Wallis and Mann-whitney non parametric rank test. Data presented are from technical duplicates. (B) ELISA-assessed neutralizing titer of spike-specific Ab against several SARS-CoV-2 variants on week 15, 19 and 22. Each curve represents an animal. Differences between groups were compared using the Kruskal-Wallis and Mann-whitney non parametric rank test.

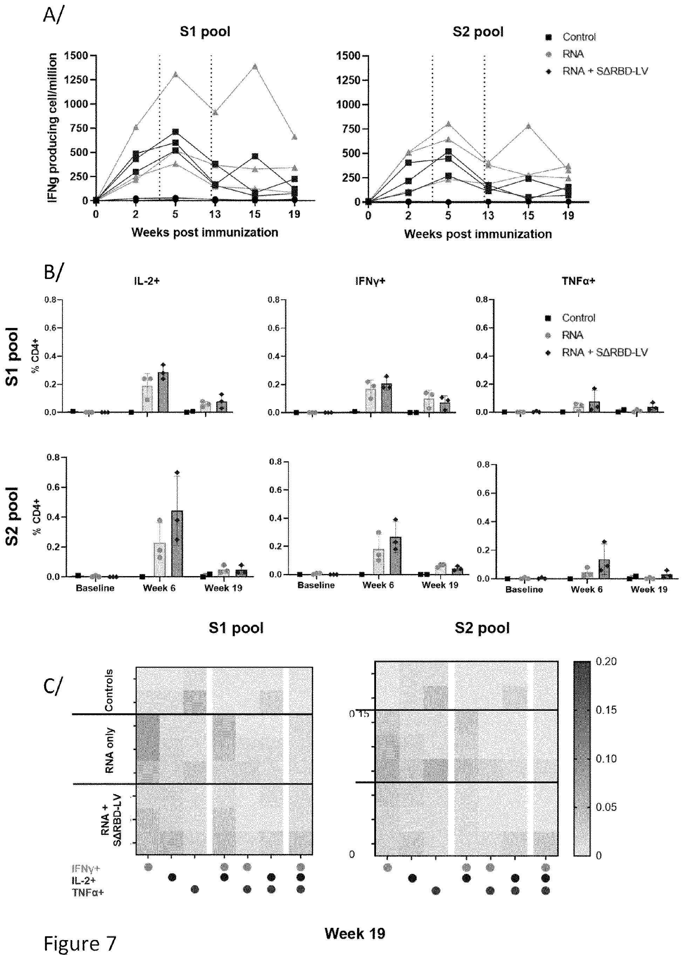

Figure 7 shows SΔRBD-LV immunization elicits a potent cellular response. (A) Spot-forming units detected by IFNy ELISPOT in peripheral blood mononuclear cells drawn on week 0, 2, 5, 13, 15 and 19 weeks after vaccination and stimulated with peptides spanning SARS-CoV-2 spike protein for 24 h. Data are represented as mean and ± SD. Differences between matched groups were compared using the Kruskal-Wallis and Mann whitney non parametric rank test. Data presented are from technical duplicates. (B) Flow cytometry characterization of T cell response from baseline, week 6 and 19 after immunization. Frequency of IFNγ+, IL-2+ and TFNα+ antigen-specific CD4+ T cells in the total CD4+ T cell population. PBMCs were stimulated overnight with SARS-CoV-2 S overlapping peptide pools. Bars indicated means. Each dot represent a single animal. Data are represented as mean and ± SD. Differences between matched groups were compared using the Kruskal-Wallis and Mann-whitney non parametric rank test. (C) Th-1 polyfunctionality of T-cells stimulated with the S1 or S2 pools was assessed 8 weeks after the third immunization. Left columns, monofonctionality, respectively IFN-y+, IL-2+ and TNFα+ single producing cells amongst CD4+ T cells, center columns bifunctionality, respectively IFN-γ+/IL-2+, IFN-γ+/TNFα+ and IL-2+/TNFα+. Right columns is complete polyfunction IFN-γ+/IL-2+/ TNFα+.

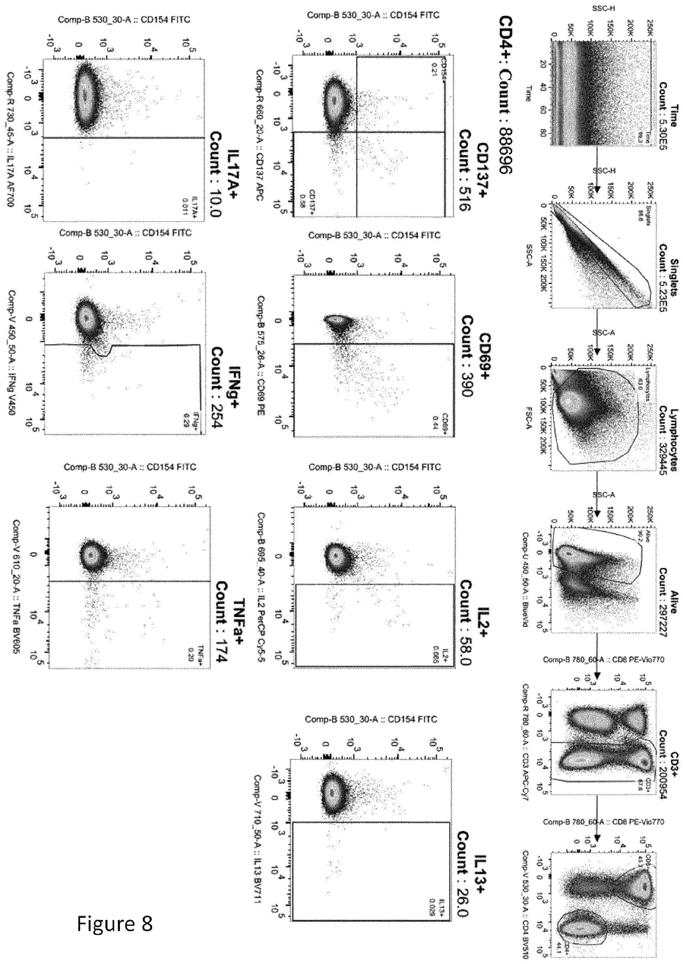

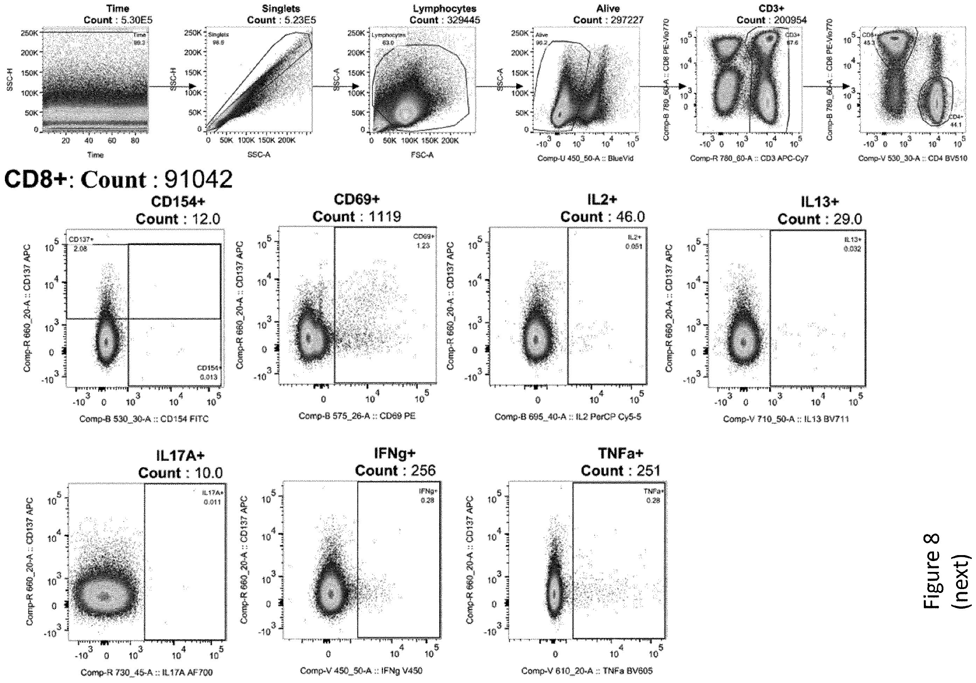

Figure 8 shows the flow cytometry intracellular gating strategy. Stimulation-specific cytokine production by CD4+ and CD8+ T cells was characterized by flow cytometry using intra-cellular straining. Cells were gated using the following approach, with each gate as a result of the previous one: singulets>lymphocytes>live cells>CD3+ cells. Amongst those cells, CD4+CD8- and CD8+CD4- were gated. In each cell type, cytokine positive cells selected, regardless of the activation status. The analysis was done using FlowJov 10 software.

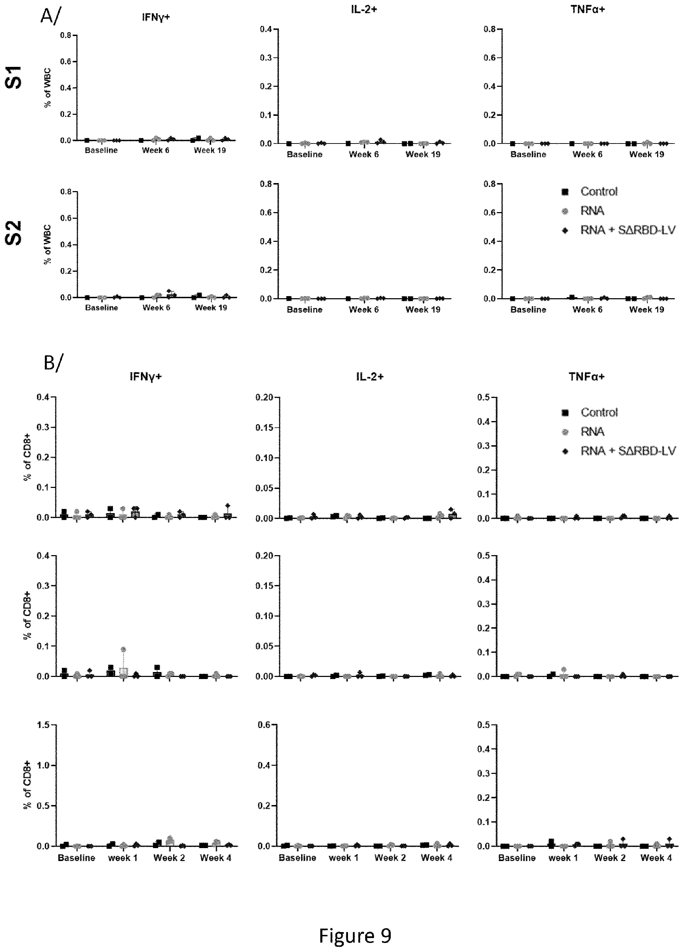

Figure 9 shows the CD8 T cell activation. CD8 T cell activation was assessed at the immunization phase (A) and after challenge (B) using flow cytometry. No cytokine production could be observed regardless of the stimulating peptide pool or group.

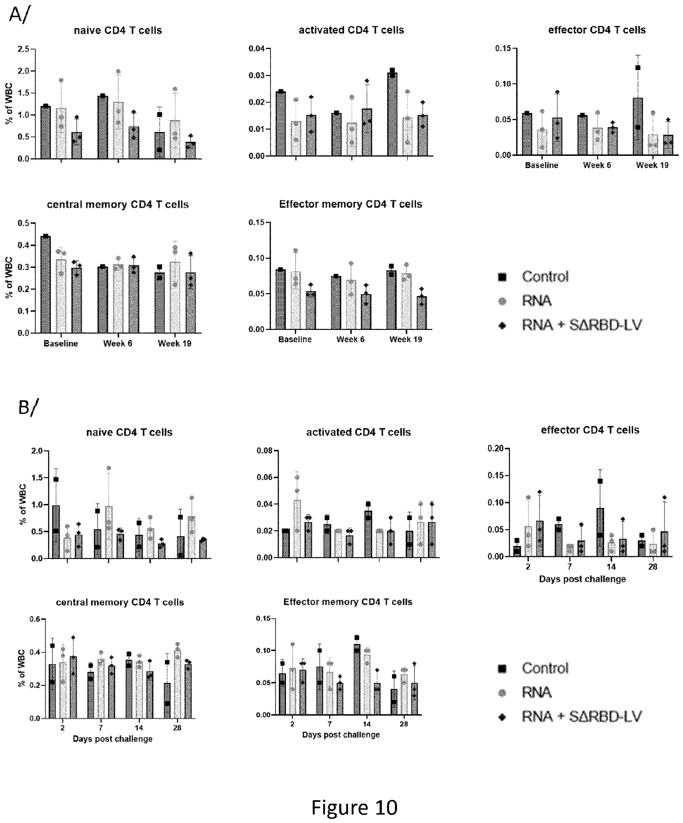

Figure 10 shows the CD4T cells subtypes. CD4T cells subtypes were characterized using flow cytometry at the immunization phase (A) and after challenge (B). Naïve T cells were gated as CD4+CD45RA+CCR7+; central memory T cells as CD4+CCR7+CD45RA-; effector cells as CD4+CCR7-CD45RA+; effector memory as CD4+ CCR7-CD45RA-; activated T cells as CD4+HLA-DR+CD69+.

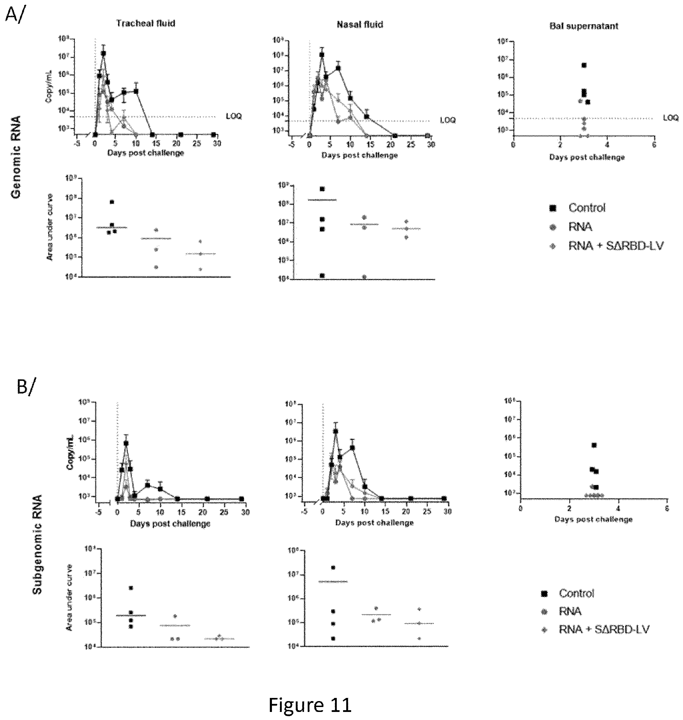

Figure 11 shows the protection from SARS-CoV-2 challenge in vaccinated cynomolgus macaques. Nasopharyngeal, tracheal and broncho-alveolar SARS-CoV-2 viral loads. Genomic (A) and subgenomic (sg, B) RNA viral loads in fluids. In each case, top row indicate viral burden measures by PCR while the lower row indicated the AUC calculated for each condition in tracheal swabs (left) and nasal swabs (middle) and broncho-alveolar fluids (right) of control (black) and RNA vaccinated (green) and RNA vaccinated, SΔRBD boosted (blue) macaques after challenge. Bars indicate mean viral loads. Vertical dotted lines indicate the day of immunization. Data are represented as mean and ± SD. Data presented are means from technical duplicates. The dotted line represent the lower limit of quantification (LOQ) when applicable.

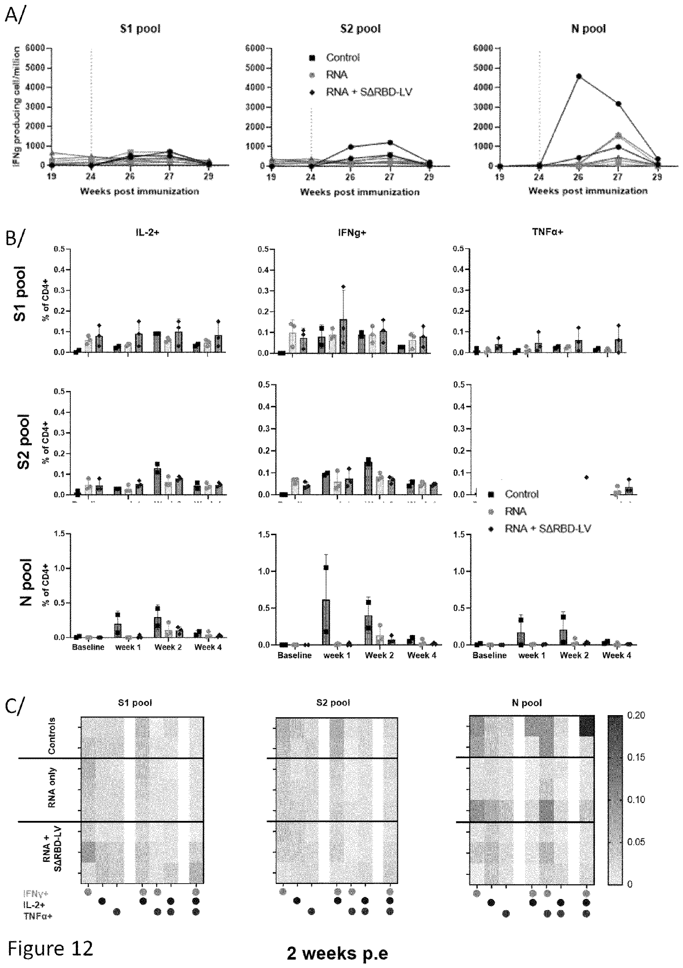

Figure 12 shows the cellular response in SARS-CoV-2 challenged macaques. (A) Spot-forming units detected by IFNy ELISPOT in peripheral blood mononuclear cells drawn before challenge and 1, 2 and 4 weeks (i.e week 24, 25, 26 and 28 after the first immunization) post exposure with XBB.1 variant. Cells were stimulated with peptides spanning SARS-CoV-2 spike protein for 24 h. Data are represented as mean and ± SD. Differences between m groups were compared using the Kruskal-Wallis non parametric rank test of Area Under Curve tests but none were significant. Data presented are from technical duplicates. (B) Flow cytometry characterization of T cell response from baseline, week 6 and 19 after immunization. Frequency of IFNy+, IL-2+ and TFNα+ antigen-specific CD4+ T cells in the total CD4+ T cell population. PBMCs were stimulated overnight with SARS-CoV-2 S overlapping peptide pools. Bars indicated means. Each dot represent a single animal. Data are represented as mean and ± SD. Differences between matched groups were compared using the Kruskal-Wallis rank test. (C) Th-1 polyfunctionality of T-cells stimulated with the S1 or S2 pools was assessed 8 weeks after the third immunization. Left columns, monofonctionality, respectively IFN-γ+, IL-2+ and TNFα+ single producing cells amongst CD4+ T cells, center columns bifunctionality, respectively IFN-y+/IL-2+, IFN-γ+/TNFα+ and IL-2+/TNFα+. Right columns is complete polyfunction IFN-γ+/IL-2+/ TNFα+.

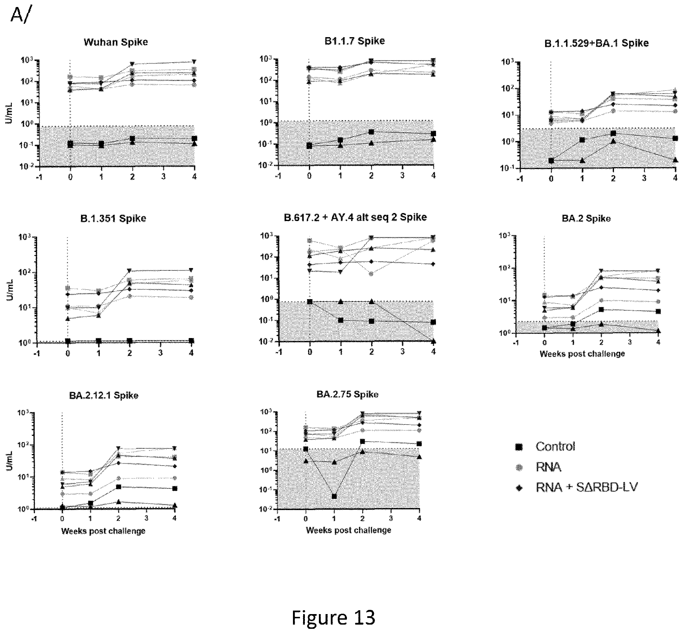

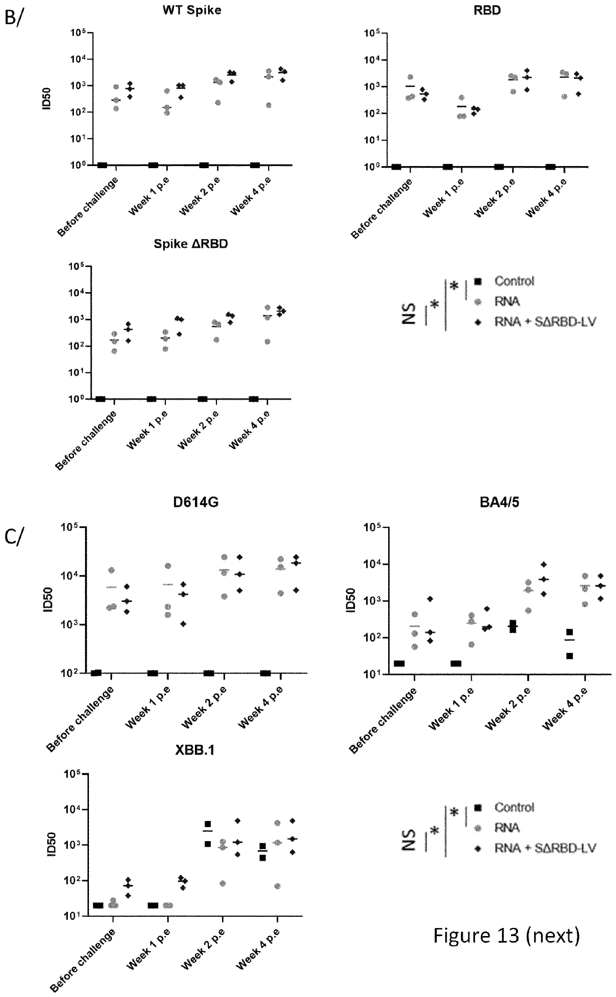

Figure 13 shows the neutralizing humoral response in challenged macaques. (A) Neutralizing titer evolution of spike-specific Ab against several SARS-CoV-2 variants before challenge and 1, 2 and 4 weeks after challenge. Each curve represents an animal. Differences between groups were compared using the Kruskal-Wallis signed-rank. (B) ELISA of SARS-CoV-2 S-protein-specific IgG determined during the study before challenge and 1, 2 and 4 weeks after challenge. Ab titers of individual animals are shown. Differences between matched groups were compared using the Kruskal-Wallis and Mann-whitney non parametric rank test. (C) Various SARS-CoV-2 strain virus neutralization are shown for sera collected at weeks 11 and 15. Bars indicate mean titers of the three animals. Neutralization is expressed as inhibitory dilution at which 50% neutralization is achieved. Differences between groups were compared using the Kruskal-Wallis and Mann-whitney non parametric rank test. Data presented are from technical duplicates.

### EXAMPLES

### Materials & Methods

### Cells lines

HEK293T (ATCC CRL-11268) {35} and HEK293F (Thermo Fisher Scientific) and are human embryonic kidney cell lines. HEK293F cells are adapted to grow in suspension. HEK293F cells were cultured at 37°C with 8% CO2 and shaking at 125 rpm in 293FreeStyle expression medium (Life Technologies). HEK293T cells were cultured at 37°C with 5% CO2 in flasks with DMEM supplemented with 10% fetal bovine serum (FBS), streptomycin (100 µg/mL) and penicillin (100 U/mL). HEK293T/ACE2 cells {36} are a human embryonic kidney cell line expressing human angiotensin-converting enzyme 2. HEK293T/ACE2 cells were cultured at 37°C with 5% CO2 in flasks with DMEM supplemented with 10% FBS, streptomycin (100 µg/mL) and penicillin (100 U/mL). VeroE6 cells (ATCC CRL-1586) are a kidney epithelial cells from African green monkeys. VeroE6 cells were cultured at 37°C with 5% CO2 in DMEM supplemented with or without streptomycin (100 µg/mL) and penicillin (100 U/mL) and with or without 5 or 10% FBS, and with or without TPCK-trypsin. PBMC were isolated from macaque sera and cultured in RPMI1640 Glutamax+ medium (Gibco^{®}) supplemented with 10 % FBS.

### Viruses

SARS-CoV-2 virus (XBB.3, a BA2.75 subvariant) was isolated by the National Reference Center for Respiratory Viruses (Institut Pasteur, Paris, France) as previously described {37} and produced by two passages on Vero E6 cells in DMEM (Dulbecco's Modified Eagles Medium) without FBS, supplemented with 1% P/S (penicillin at 10,000 U ml-1 and streptomycin at 10,000 µg ml-1) and 1 µg ml-1 TPCK-trypsin at 37 °C in a humidified CO2 incubator and titrated on Vero E6 cells. Whole genome sequencing was performed as described {37} with no modifications observed compared with the initial specimen and sequences were deposited after assembly on the GISAID EpiCoV platform under accession number ID EPI_ISL_410720. The SARS-CoV-2 hCoV-19/France/HDF-IPP53307/2022, Omicron, lineage XBB.3 is now available at https://www.european-virus-archive.com under Ref-SKU: 017V-04974.

### Ethics and biosafety statement

Cynomolgus macaques (Macaca fascicularis) originating from Mauritian AAALAC certified breeding centers were used in this study.

| | vaccine | Gender | Date of birth | Age (years) | Weight at Day 0 post exposure (kg) | Dev. stage |
|---|---|---|---|---|---|---|
| CHE040 | / | F | 24/05/2018 | 4.8 | 3.2 | Young adult |
| 1KJ17 | / | M | 06/04/2017 | 5.9 | 7.8 | Young adult |
| CHH062 | / | M | 31/08/2018 | 4.5 | 5.9 | Young adult |
| BC219 | / | F | 12/12/2006 | 16.1 | 4,89 | Mature adult |
| BY647 | / | F | 11/07/2010 | 12.5 | 4,84 | Mature adult |
| CH042 | Comirnaty | F | 25/05/2018 | 4.8 | 3.5 | Young adult |
| CIB033 | Comirnaty | F | 06/02/2019 | 4.1 | 3.4 | Young adult |
| CIB002 | Comirnaty | F | 02/02/2019 | 4.1 | 3.8 | Young adult |
| CIA044 | SΔRBD | F | 22/01/2019 | 4.1 | 3.67 | Young adult |
| DB445 | SΔRBD | F | 07/09/2017 | 5.5 | 5.13 | Young adult |
| DF933 | SΔRBD | M | 19/09/2018 | 4.5 | 5.15 | Young adult |

All animals were housed in IDMIT infrastructure facilities (CEA, Fontenay-aux-roses), under BSL-2 and BSL-3 containment when necessary (Animal facility authorization #D92-032-02, Préfecture des Hauts de Seine, France) and in compliance with European Directive 2010/63/EU, the French regulations and the Standards for Human Care and Use of Laboratory Animals, of the Office for Laboratory Animal Welfare (OLAW, assurance number #A5826-01, US). The protocols were approved by the institutional ethical committee "Comité d'Ethique en Expérimentation Animale du Commissariat à I'Energie Atomique et aux Energies Alternatives" (CEtEA #44) under statement number A20_061. The study was authorized by the "Research, Innovation and Education Ministry" under registration number APAFIS#28946-2021011312169043 v2.

### Animals and study design

Cynomolgus macaques were randomly assigned in three experimental groups. One Comirnaty vaccinated group (n = 3) received three doses of the Pfizer/BioNTech Comirnaty (30 µg, Comirnaty group) and the second group (n=3) received two doses of Comirnaty and a third dose of 50 µg of SΔRBD-LVs adjuvanted with 250 µg of MPLA liposomes (Polymun Scientific, Klosterneuburg, Austria) diluted in PBS at weeks 0, 4 and 11, while control animals (n = 4) two received only PBS and two nothing. Vaccinated animals were sampled in blood at weeks -2, 0, 2, 4, 6, 11, 13, 15, 19, 21 and 23. At week 24, all animals were exposed to a total dose of 105 pfu of SARS-CoV-2 virus (XBB.3 strain) via the combination of intranasal and intra-tracheal routes (0,25 mL in each nostril and 4,5 mL in the trachea, i.e., a total of 5 mL; day 0), using atropine (0.04 mg/kg) for pre-medication and ketamine (5 mg/kg) with medetomidine (0.042 mg/kg) for anesthesia. Nasopharyngeal and tracheal swabs, were collected at days 1, 2, 3, 4, 7, 10, 14 and 29 days past exposure (dpe) while blood was taken at days 2, 4, 7, 10, 14 and 29 dpe. Bronchoalveolar lavages (BAL) were performed using 50 mL sterile saline on 3 dpe. Blood cell counts, hemoglobin, and hematocrit, were determined from EDTA blood using a DHX800 analyzer (Beckman Coulter).

### Protein expression and purification

The gene of nucleotide sequence SEQ ID No: 2, encoding for a protein/protomer of amino acid sequence SEQ ID No: 1, corresponding to residues 1-331-GSGSGS-530-1208 (RBD residues 332-529 deleted) pf the native SARS-CoV-2 S gene (Wuhan-Hu-1 Spike) with proline substitutions at residues K986P, V987P, F817P, A892P, A899P, A942P ("6P"), a "GSAS" (SEQ ID No: 3) substitution at the furin cleavage site (residues 682-685) a C-terminal T4 fibritin trimerization motif (SEQ ID No: 4) and a linker GSGHHHHHHHHGSGC (SEQ ID No: 5) containing 8xHis-tag {38; 39} was transiently expressed in FreeStyle293F cells (Thermo Fisher scientific) using polyethylenimine (PEI) 1 µg/µl for transfection. Supernatants were harvested five days post-transfection, centrifuged for 30 min at 5000 g and filtered using 0.45 µm filters (ClearLine^{®}). SARS-CoV-2 S protein was purified from the supernatant by Ni2+-Sepharose chromatography (Excel purification resin, Cytiva) in buffer A (50 mM HEPES pH 7.4, 200 mM NaCl) and eluted in buffer B (50 mM HEPES pH 7.4, 200 mM NaCl, 500 mM imidazole). Eluted SARS-CoV-2 S containing fractions were concentrated using Amicon Ultra (cut-off: 30 KDa) (Millipore) and further purified by size-exclusion chromatography (SEC) on a Superose 6 column (GE Healthcare) in buffer A or in PBS. The SARS-CoV-2 S RBD domain (residues 319 to 541) was expressed in EXPI293 cells by transient transfection according to the manufacturer's protocol (Thermo Fisher Scientific) ad as described {40}. Briefly, supernatants were harvested five days after transfection and cleared by centrifugation. The supernatant was passed through a 0.45 µm filter and RBD was purified using Ni2+-chromatography (HisTrap HP column, GE Healthcare) in buffer C (20 mM Tris pH 7.5 and 150 mM NaCl buffer) followed by a washing step with buffer D (20 mM Tris pH 7.5 and 150 mM NaCl buffer, 75 mM imidazole) and elution with buffer E (20 mM Tris pH 7.5 and 150 mM NaCl buffer, 500 mM imidazole). Eluted RBD was further purified by SEC on a Superdex 75 column (GE Healthcare) in buffer C. Protein concentrations were determined using an absorption coefficient (A1%,1cm) at 280 nm of 10.4 and 13.06 for S protein and RBD, respectively, using ProtParam (https://web.expasy.org/).

### Trimer crosslinking

The trimer "SΔRBD protein" at 1 mg/ml in PBS was cross-linked with 4% formaldehyde (FA) (Sigma) overnight at room temperature. The reaction was stopped with 1 M Tris HCl pH 7.4 adjusting the sample buffer to 7.5 mM Tris/HCl pH 7.4. FA was removed by PBS buffer exchange using 30 KDa cut-off concentrators (Amicon). FA crosslinking was confirmed by separating the formaldehyde treated trimer "FA-SΔRBD" on a 10% SDS-PAGE under reducing conditions.

### Trimer coupling to liposomes

Liposomes for conjugating S protein were prepared as described previously {40}. Briefly, liposomes were composed of 60% of L-α-phosphatidylcholine, 4% His tag-conjugating lipid, DGS-NTA-(Ni2+) and 36% cholesterol (Avanti Polar Lipids). Lipid components were dissolved in chloroform, mixed and placed for two hours in a desiccator under vacuum at room temperature to obtain a lipid film. The film was hydrated in filtered (0.22 µm) PBS and liposomes were prepared by extrusion using membrane filters with a pore size of 0.1 µm (Whatman Nuclepore Track-Etch membranes). The integrity and size of the liposomes was analyzed by negative staining-EM. For protein coupling, the liposomes were incubated overnight with FA-SΔRBD or SΔRBD protein in a 3:1 ratio (w/w). Free FA-SΔRBD protein was separated from the FA-SΔRBD-proteoliposomes (SΔRBD-LVs) by sucrose gradient (5-40%) centrifugation in a SW55 rotor at 40,000 rpm for 2 h. The amount of protein conjugated to the liposomes was determined by Bradford assay and SDS-PAGE densitometry analysis comparing SΔRBD-LV bands with standard FA-SΔRBD protein concentrations.

### Trimer thermostability

Thermal denaturation of native SARS-CoV-2 S '6P', SΔRBD and FA-SΔRBD was analyzed by differential scanning fluorimetry (DSF) coupled to back scattering using a Prometheus NT48 instrument (Nanotemper Technologies, Munich, DE). Protein samples were first extensively dialyzed against PBS pH 7.4, and the protein concentration was adjusted to 0.3 mg/ml. 10 µL of sample were loaded into the capillary and intrinsic fluorescence was measured at a ramp rate of 1°C/min with an excitation power of 30 %. Protein unfolding was monitored by the changes in fluorescence emission at 350 and 330 nm. The thermal unfolding midpoint (Tm) of the proteins was determined using the Prometheus NT software.

### Negative stain electron microscopy

Protein samples were visualized by negative-stain electron microscopy (EM) using 3-4 µL aliquots containing 0.1-0.2 mg/ml of protein. Samples were applied for 10 s onto a mica carbon film and transferred to 400-mesh Cu grids that had been glow discharged at 20 mA for 30 s and then negatively stained with 2% (wt/vol) Uranyl Acetate (UAc) for 30 s. Data were collected on a FEI Tecnai T12 LaB6-EM operating at 120 kV accelerating voltage at 23k magnification (pixel size of 2.8 Å) using a Gatan Orius 1000 CCD Camera. Preliminary 2-D class averaging was performed with Relion {41} using 9800 particles, a pixel size of 3.9 Å/pixel and a box size of 200 pixels.

### Cryo-electron microscopy

*Data collection.* 3.5 µL of SΔRBD sample were applied to 1.2/1.3 C-Flat (Protochips Inc) holey carbon grids or 1.2/1.3 Ultrafoil grids (Quantifoil MicroTools GmbH, Germany) and plunged frozen in liquid ethane with a Vitrobot Mark IV (Thermo Fisher Scientific) (6 s blot time, blot force 0). The sample was observed at the beamline CM01 of the ESRF (Grenoble, France) {42} with a Titan Krios G3 (Thermo Fischer Scientific) at 300 kV equipped with an energy filter (Bioquantum LS/967, Gatan Inc, USA) (slit width of 20 eV). Movies were recorded automatically on two different grids with a K3 direct detector (Gatan Inc., USA) with EPU (Thermo Fischer Scientific). A total of 11051 movies were recorded for a total exposure time of 1.6 s with 40 frames per movie and a total dose of ^{~}40 e-/Å2. The magnification was 105,000x (0.84 Å/pixel at the camera level). The defocus of the images was varied between -1.0 and -2.5 µm.

*3D reconstruction.* The movies were first drift-corrected with motioncor2 {43}. CTF estimation was done with GCTF {44}. The remaining image processing was performed with RELION 4 {45}. An initial set of particles (box size of 86 pixels, sampling of 3 Å/pixel) was obtained by auto-picking. After two rounds of 2D classification, the particles from the best looking 2D class averages were used to generate an ab-initio 3D map (with C1 symmetry) which was then refined to 6.2 Å imposing C3 symmetry. The 3D map displays the features expected for SΔRBD and an atomic model of S could un-ambiguously be docked in the map. The particles used to generate that first 3D map were then used to train a picking model and pick all the micrographs with Topaz {46}. Approximately 1.1 M of particles were extracted and a first 3D classification (C1 symmetry, 5 classes and a circular mask diameter of 210 Å ) allowed the identification of a subset of 200,114 particles which were then re-extracted (box size of 400 pixels, sampling of 0.84 Å/pixel). A 3D refinement (C3 symmetry) gave a 3D reconstruction at 4 Å resolution. Refinement of CTF parameters, particle polishing and a second round of CTF parameter's refinement further improved the resolution to 3.1 Å. A final 3D classification (C1 symmetry, 3 classes and a circular mask diameter of 210 Å) allowed the identification of a subset of 87,324 particles which then yielded the final 3D reconstruction at 3.0 Å resolution. The resolution was determined by Fourier Shell Correlation (FSC) at 0.143 between two independent 3D maps. The local resolution was calculated with blocres {47} and found to be between 2.8 and 4.4 Å. The final 3D map was sharpened with DeepEMhancer for display purpose {48}.

*Model refinement.* The atomic model of the S protein, paraformaldehyde fixed, in the closed conformation (PDB 7Q1Z) {40} was rigid-body fitted inside the cryo-EM density map in CHIMERA {49}. The residues corresponding to the RBD domains were deleted. The atomic coordinates were then refined with Rosetta {50} and PHENIX {51}. The refined atomic models were visually checked and adjusted (if necessary) in COOT {52}. The final model was validated with MOLPROBITY {53}. The figures were prepared with CHIMERA and CHIMERAX {49; 54}. The atomic coordinates and the cryo-EM map have been deposited in the Protein Data Bank and in the Electron Microscopy Data Bank under the accession codes 8R87 and EMD-18997, respectively.

### Virus quantification in NHP samples

Upper respiratory (nasopharyngeal and tracheal) specimens were collected with swabs (Viral Transport Medium, CDC, DSR-052-01). Tracheal swabs were performed by insertion of the swab above the tip of the epiglottis into the upper trachea at approximately 1.5 cm of the epiglottis. All specimens were stored at - 80°C until extraction and analysis. Extraction was done using the NucleoSpin^{™} Virus Core Kit (Macherey-Nagel, Dueren, Germany) according to the manufacturer's instruction. Samples were then analyzed by RT-qPCR with a virus standard concentration range containing an RdRp gene fragment including the RdRp-IP4 RT-PCR target sequence IP4-SARS-CoV-2-R CTG GTC AAG GTT AAT ATA GG (SEQ ID No: 6), IP4-SARS-CoV-2--F: GGT AAC TGG TAT GATTTC G (SEQ ID No: 7) and SARS6CoV-2 IP4 probe: FAM-TCA TAC AAA CCA CGC CAG G-BHQ1 (SEQ ID No: 8). SARS-CoV-2 E gene subgenomic mRNA (sgRNA) levels were assessed by RT-qPCR using primers and probes previously described {55; 56}:
- leader-specific primer sgLeadSARSCoV2-F of sequence : CGATCTCTTGTAGATCTGTTCTC (SEQ ID No: 9),
- E-Sarbeco-R primer of sequence ATATTGCA GCAGTACGCACACA (SEQ ID No: 10) and
- E-Sarbeco probe of sequence HEX-ACACTAGCCATCCTTACTGCGCTTCG-BHQ1 (SEQ ID No: 11), wherein HEX represents hexachlorofluorescein and BHQ1 represents the BHQ1 quencher.

The protocol describing this procedure for the detection of SARS-CoV-2 is available on the WHO website (https://www.who.int/docs/default-source/coronaviruse/whoinhouseassays.pdf). RT-qPCR was performed using the SuperScript^{®} III Platinium^{®} One-step Quantitative RT-qPCR System kit (Life Technologies, CA, USA) and C1000 Thermal Cylcer with CF96 optic module (Bio-Rad Laboratores, Diamed GmbH, Switzerland) according to the manufacturer's instruction. Data were then analyzed using CFX Maestro (v2.2, Bio-Rad).

### ELISA

Serum antibody titers specific for soluble WT S glycoprotein, SΔRBD and for RBD were determined using an enzyme-linked immunosorbent assay (ELISA). Briefly, 96-well micro titer plates (F96 Maxisorp NUNC Immunoplate #442404) were coated with 50 µl at 1 µg/ml of WT S, SΔRBD or RBD proteins at 4°C overnight in PBS and blocked with 3% BSA for 1 h at room temperature after 3 washes with PBS Tween-20 0.05 %. Serum dilutions were added to each well and plates were washed 5 times with PBS Tween. An alkaline phosphatase conjugated anti-monkey IgG (Sigma Aldrich, # A1929)was then diluted 1/10000 and incubated for 1h, before excess Ab was washed out and PNPP substrate added (Interchim, # UP 664791) . Absorbance was read at 1h15, at 405 nm. Antibody titers were expressed as ED50 (Effective Dilution 50-values) and were determined as the serum dilution at which IgG binding was reduced by 50%. ED50 were calculated from crude data (O.D) after normalization using GraphPad Prism (version 9) "log(inhibitor) vs normalized response" function.

### Pseudovirus neutralization assay

Neutralization activity was tested using a pseudovirus neutralization assay, as previously described {35}. Shortly, pseudoviruses were produced by co-transfecting the pCR3 SARS-CoV-2-SΔ19 (Wuhan-Hu-1, GenBank MN908947.3 with amino acid substitution D614G) expression plasmid with the pHIV-1NL43 ΔEnv-NanoLuc reporter virus plasmid in HEK293T cells (ATCC, CRL-11268), as previously described {36}. Cell supernatant containing the pseudovirus was harvested 48 hours post transfection and stored at -80°C until further use. For the neutralization assay itself, HEK293T/ACE2 cells {36} were seeded at a density of 20,000 cells/well in a 96-well plate coated with 50 µg/mL poly-L-lysine one day prior to the start of the neutralization assay. Antibodies were serially diluted in cell culture medium (DMEM (Gibco), supplemented with 10% FBS, penicillin (100 U/mL), streptomycin (100 µg/mL) and GlutaMax (Gibco)), subsequently mixed in a 1:1 ratio with pseudovirus and incubated for 1 hour at 37°C. Next, these mixtures were added to the cells in a 1:1 ratio and incubated for 48 hours at 37°C, followed by cell lysis to measure the luciferase activity in cell lysates using the Nano-Glo Luciferase Assay System (Promega) and GloMax system (Turner BioSystems). Relative luminescence units (RLU) were normalized to the positive control wells where cells were infected with pseudovirus in the absence of antibodies. The neutralization titers (IC50) were determined as the antibody concentration at which infectivity was inhibited by 50%, respectively, using a non-linear regression curve fit (GraphPad Prism software version 8.3).

### Electrochemiluminescence-based neutralizing antibody assay.

Neutralizing antibody titers were measured using the Meso Scale Discovery MSD V-PLEX SARS-CoV-2 Key Variant Spike Panel 1 ACE2 competition assay kit according to the manufacturer's instructions. This technique permits to measure the activity of antibodies capable of blocking the binding of ACE2 to its cognate receptor from the Wuhan virus and from (B.1.1.7), (B.1.1.529; BA.1), (B.1.351), (B.1.617.2; AY.4) Alt Seq 2, (BA.2), (BA.2.12.1), (BA.2.75), and (BA.5) variants. Plates were blocked with MSD Blocker A and washed.The assay calibrator (COVID-19 neutralizing antibody; monoclonal antibody against S protein), control sera and test sera samples diluted 1 in 10 or 1 in 100 in assay diluent were added to the plates. Following a 2h incubation period, the plates were washed, MSD SULFO-TAG^{™} conjugated ACE-2 protein was added. The plates were then read using a MESO^{®} QuickPlex SQ 120MM Reader. The calibration curve was used to calculate neutralizing antibody concentrations in samples by backfitting the measured electrochemiluminescence (ECL) signals to the calibration curve. Data analysis was conducted using the Discovery Workbench software (v4.0 MSD, Rockville USA) and the results were expressed as units (U)/mL.

### Antigen specific IFNγ producing T cell assays using ELISpot

Monkey IFN-y ELISpot PRO kit (Mabtech Monkey IFN-y ELISpot pro, #3421M-2APT) was used following manufacturer's instructions. 2 × 105 freshly isolated PBMCs were added to each well. Cells were stimulated using SARS-CoV-2 peptid pools in the culture medium at a final dilution of 2 µg/mL. Before challenge, 2 pools of 158 and 157 peptides derived from a peptide scan through the Spike glycoprotein of SARS-CoV-2 were used. After challenge, an additional pool of 102 peptides derived from a peptide scan through Nucleoprotein was used. PMA/ionomycine (62,5ng/mL and 714,29ng/mL respectively) was used as positive control and complete RPMI medium alone as negative control. Plates were incubated for 18 hours at 37 °C in an atmosphere containing 5 % CO2 then washed 5 times with PBS. Alkaline phosphatase linked anti-IFN-y antibody was added and the plates were incubated 2 hours at 37 °C. Plates were washed 5 times with PBS and spots were developed by adding 0.45 µm-filtered ready-to-use BCIP/NBT-plus substrate solution. The spots were counted with an Automated IRIS^{™} FluoroSpot/ELISpot Reader (Mabtech AB, Stockholm, Sweden) at CEA.

### Antigen specific T cell assays using flow cytometry

To analyze the SARS-CoV-2 protein-specific T cell, 15-mer peptides (n = 157 and n=158) overlapping by 11 amino acids (aa) and covering the SARS-CoV-2 Spike sequence (aa 1 to 1273) were synthesized by JPT Peptide Technologies (Berlin, Germany) and used at a final concentration of 2 µg/mL.

T-cell responses were characterized by measurement of the frequency of CD4+ T cells expressing IL-2 (PerCP5.5, MQ1-17H12, BD), IL-17a (Alexa700, N49-653, BD), IFN-y (V450, B27, BD), TNF-α (BV605, Mab11, BioLegend), IL-13 (BV711, JES10-5A2, BD), CD137 (APC, 4B4, BD), CD69 (PE-, FN-50, BioLegend) and CD154 (FITC, TRAP1, BD) upon stimulation with the two peptide pools. CD3 (APC-Cy7, SP34-2, BD), CD4 (BV510, L200, BD) and CD8 (PE-Vio770, BW135/80, Miltenyi Biotec) antibodies was used as lineage markers. One million of PBMC were cultured in complete medium (RPMI1640 Glutamax+, Gibco; supplemented with 10 % FBS), supplemented with co-stimulatory antibodies (Fastlmmune CD28/CD49d, Becton Dickinson). The cells were stimulated with S sequence overlapping peptide pools at a final concentration of 2 µg/mL. Brefeldin A was added to each well at a final concentration of 10 µg/mL and the plate was incubated at 37°C, 5% CO2 during 18 h. Next, cells were washed, stained with a viability dye (LIVE/DEAD fixable Blue dead cell stain kit, ThermoFisher), and then fixed and permeabilized with the BD Cytofix/Cytoperm reagent. Permeabilized cell samples were stored at -80 °C before the staining procedure. Antibody staining was performed in a single step following permeabilization. After 30 min of incubation at 4°C, in the dark, cells were washed in BD Perm/Wash buffer then acquired on the LSRII cytometer (Beckton Dickinson). Analyses were performed with the FlowJo v.10 software. Data are presented a minus background where non-stimulated cells signals was subtracted to each specific signal.

### Statistical analysis

Statistical significance between groups was performed using Graphpad Prism (v9.2.0). Statistical analysis of NHP gRNA, sgRNA and Ab production were carried out using Kruskal-Wallis and Mann-Whitney unpaired t-test in GraphPad Prism software (v9.2.0).

### Results

### SARBD-LV formation and characterization

The S glycoprotein construct '6P' {39} without RBD (SΔRBD) was expressed in mammalian cells and purified by Ni2+-affinity and size exclusion chromatography (SEC) (Figure 2A). Because SΔRBD showed a Tm of 44.5°C in comparison to 63.4 °C for S '6P', SΔRBD was stabilized by chemical cross-linking with 4% formaldehyde (FA). This produced a high molecular weight species as determined by SDS-PAGE (Figure 2A). FA-SΔRBD revealed an increased Tm of 56.3 °C indicating successful stabilization by cross-linking. Negative staining electron microscopy analyses demonstrated the native prefusion conformation of FA-SΔRBD (Figure 2B) and its structure was subsequently solved at 3.2 Å resolution by cryo-electron microscopy confirming the prefusion S conformation (Figures 1A, 3). The structure revealed one major cross-linking site between residues R1019 of the central S2 helix and/or S2 K776 and S2 HR1 K947 from neighboring protomers (Figures 1A and B), which stabilized S in the prefusion conformation. FA SΔRBD was incubated with liposomes (Phosphatidylcholine 60%, cholesterol 36%, DGS-NTA 4%), and efficiently captured via its C-terminal His-tag. Free, unbound FA-SΔRBD was removed from the FA-SΔRBD proteoliposomes by sucrose gradient centrifugation (Figure 2C) and decoration of the liposomes with FA-SΔRBD (SΔRBD-LV) was confirmed by negative staining electron microscopy (Figure 2D).

### SΔRBD-LVs immunization induced potent neutralizing antibody responses in cynomolgus macaques

To assess the efficacy of SΔRBD as a booster candidate, we immunized one group (n=3) of cynomolgus macaques three times (at weeks 0, 4, 11) with the Pfizer/BioNTech Comirnaty vaccine (Comirnaty group). Another group (n=3) received two doses of Comirnaty followed by a SΔRBD-LV boost (SΔRBD group) adjuvanted with monophospholipid A (MPLA) liposomes by the intramuscular route (Figure 4A). No weight loss was observed and no abnormality was recorded in the blood cellular formula during the 8 weeks following the immunization of the animals (Figure 5). The sera of the immunized macaques were analyzed for binding to native the S glycoprotein (S), to the RBD and to the SΔRBD. This analysis showed similar S-specific Ab titers for all vaccinated animals.

The ED50 titers for S, RBD and SΔRBD are similar at week 4 and week 11 with slight variations between animals, since both groups were immunized twice with Comirnaty. In general ED50 titers increased from increased by ^{~}100 times from week 4 to ^{~}1000 on week 11 after the second immunization (Figure 4B). After the third immunization ED50 S titers increased in the Comirnaty group and stayed stable in the S SΔRBD group. ED50 RBD titers were slightly lower in the SΔRBD group compared to the Comirnaty group, while the SΔRBD titers increased in both groups. ED50 titers against wild type S remained stable for the SΔRBD-LV group but reached a peak augmentation of 13210 times the initial titer on week 15 before diminishing in the Comirnaty group (Figure 4B). Ab titers decreased in both groups on week 19, and 23 respectively 8 and 12 weeks after the third immunization as observed in many studies before, however the decrease in Ab titer against SΔRBD was less pronounced compared to the decrease in RBD-specific antibodies (Figure 4B). This suggests that SΔRBD immunization redirects the immune response to non-RBD epitopes resulting in overall similar S-specific Ab titers.

Significant serum neutralization titers were elicited in both groups after the second immunization, and these were further boosted by the third immunization. On week 15, the pseudovirus neutralization titers against the D614G mutant showed mean ID50 titers of 18188 for the Comirnaty group and 9549 for the SΔRBD group after the third immunization. Mean ID50 titers against IN2 (Delta) were 10231 and 1873 for the Comirnaty and SΔRBD groups, respectively and against Omicron variants BA1, the mean ID50 titers were 1550 and 655, and for BA4/5 they were 1340 and 1044 for both groups, respectively (Figure 6A). Neutralization titers were also assessed using the ACE-2 pseudo neutralization assay, which revealed potent neutralization against the Wuhan and B1.1.7 (Alpha) strains for both groups with sera from the Comirnaty group sera exhibiting higher potency. Neutralization against Omicron B.1.1.529/ BA.1, BA.2 and BA.2.12.1 was very weak in both groups, while neutralizing titers against B.617.2 (Delta) and BA.2.75 (Omicron) were comparable to those against the ancestral Wuhan strain (Figure 6B). The inventors conclude that one boost with the SΔRBD-LVs induces comparable neutralization profiles, albeit slightly possibly reduced for some variants, suggesting that SΔRBD-LVs activate antibodies targeting neutralization epitopes outside the RBD. *Vaccine-induced T-cell response* T-cell responses were observed in all vaccinated macaques following *ex vivo* stimulation of PBMCs with S-peptide pools, with no significant difference between the Comirnaty or SΔRBD-LV vaccinated groups (Figure 7A). The response culminated on week 5 for each vaccinated group with a peak of 736 (± 499) IFN-y producing cells per million in the Comirnaty group and 611 (± 97) for the SΔRBD group. The production reached lower stable levels on week 13 for each vaccinated group. Vaccine-specific T cell subtypes were also investigated using intracellular staining of expressed cytokines (Figure 8). After three immunization doses, specific CD4+ T cells producing mostly either IFNy or co-producing IFNy and IL-2 were observed against both the S1 and S2 peptide pools in all vaccinated animalswith no significant difference between the groups (Figures 7B and C). Furthermore, none of the groups had detectable anti-S CD8+ T cells (Figure 9) nor major T cell sublineage proportion modification (Figure 10).

### Challenge with Omicron variant

On week 24, 13 weeks after the third immunization with the Comirnaty vaccine, the SΔRBD and control (n=4) groups were challenged with a total dose of 1 × 10⁵ plaque-forming units (pfu) of the XBB.3 strain, a BA.2.75 omicron variant by combining intranasal (0.25 mL into each nostril) and intratracheal (4.5 mL) routes. Viral load in the control group peaked in the trachea 2 days post-challenge (dpe) with a mean peak viral load of 1.61.10⁷ (± 3.015.10⁷) copies per ml and an area under curve (AUC) of 1.8.10⁷ (± 3.05.10⁷). Complete clearance was observed at 14 dpe. Similar profiles were observed in both vaccinated groups. The Comirnaty group showed a peak viral burden of 1.17×10⁵ (± 1.64.10⁵) copies per ml at 2 dpe and an AUC of 2.70.10⁵ (± 3.21.10⁵). The viral load of the SΔRBD group peaked at 2 dpe with 8.43×10⁵ (± 1.28.10⁶) copies per ml at 2 dpe and an AUC of 8.92.10⁵ (± 1.31.10⁶). The SARS-CoV-2 virus remained detectable until day 14 post-challenge in both vaccinated groups (Figure 11A). Genomic RNA was also detected in nasal fluids with both vaccinated groups exhibiting similar profiles. The control group peaked at 3 dpe with 1.17×10⁸ (± 2.34.10⁸) copies per ml with an AUC of 1.72.10⁸ (±3.30.10⁸) and complete clearance at 21 dpe. Both Comirnaty and SΔRBD groups peaked at 2 dpe with mean of 3.25×10⁶ (± 5.59.10⁶) copies per mL and 3.71.10⁶ (± 4.73.10⁶) copies per mL respectively, and an AUC of 8.71.10⁶ (±1.04.10⁷) and 6.38.10⁶ (±5.36.10⁶) respectively. Complete viral clearance was observed in both groups at 14 dpe.

In the bronchoalveolar lavage (BAL), the four control animals showed significant viral loads at 3 dpe with a mean of 1.28×10⁶ (± 2.35.10⁶) copies/mL. All animals of the Comirnaty group exhibited detectable genomic RNA in the BAL even though only one had an above limit of quantification (LOQ) viral burden. The three RNA + SΔRBD-LV vaccinated animals had a below LOQ viral load with 2 of them in the limit of detection range (Figure 11A).

Viral subgenomic RNA (sgRNA), which estimates the number of infected and productively infected cells confirmed a similar behavior in both vaccinated groups. The control group showed peak copy numbers in tracheal fluids at 2 dpe with mean of 6.71×10⁵ (± 1.22.10⁶) copies/mL and an AUC of 7.65.10⁵ (±1.23.10⁶) whereas in the vaccinated groups, a viral load mean peak of 3.27×10³ (± 4.36.10³) copies/mL for the Comirnaty group and 5.53×10⁴ (± 9.45.10⁴) copies/mL for the SΔRBD group. Both vaccinated groups had consistent AUC with 2.42.10⁴ (±4.40.10³) for the Comirnaty group and 7.63.10⁴ (±9.45.10⁴) copies per mL for the SΔRBD group were observed. Similar subgenomic RNA copy numbers were detected in nasal fluids, with mean peak viral load of 3.42×10⁶ (± 6.84.10⁶) copies/mL and a AUC of 5.08.10⁶ (±9.88.10⁶) in the control group, 5.49×10⁴ (± 9.37.10⁴) copies/mL with an AUC of 1.60.10⁵ (±1.81.10⁵) in the Comirnaty and 1.13 ×10⁵ (± 1.04.10⁵) with an AUC of 2.17.10⁵ (±1.59.10⁵) in the SΔRBD groups. BAL revealed 1.12 ×10⁵ (± 2.00.10⁵) copies per mL in the control group, 1.2×10³ (± 909) copies per mL in the Comirnaty group and below the detection limit sgRNA in the SΔRBD group (Figure 11B). In summary, the detection of genomic and subgenomic RNA indicates that vaccination reduces viral load by 1 to 2 logs, although not significantly due to the low number of animals, notable variations between individuals and the non-parametric nature of the statistical tests.

Specific IFN-y producing cells in the blood were analyzed after challenge with the Omicron variant. No significant difference was observed between the vaccinated groups, both against S1- and S2-stimulation at day 14 post challenge, as previously observed in an anamnestic response (Figure 12A). Furthermore, the vaccinated animals from both groups demonstrated a limited response to the N peptide pool stimulation. In contrast, the control animals exhibited a de novo anti-S1, -S2 and -N Th1 CD4+ response 1 to 2 weeks post challenge, (Figure 12B). This response was mostly IFNy/IL-2 dependent after stimulation with S1 and S2 pools, with no difference between the two vaccinated groups. Conversely the de novo N-specific response was IFNγ/TNFα oriented in every group, with one control animal exhibiting a strong trifunctional T cell response (Figure 12C). No CD8 T cell activation was observed after challenge, regardless of the group (Figure 9).

An electrochemiluminescence (ECL)-based ACE2 competition assay employing different S variants was used to assess pseudoneutralizing potency of the antibody pool. This assay included Wuhan, BA.2.75 (omicron, subvariant of the challenge virus XBB.3), B1.1.7 (alpha), B.617.2 +Ay alt seq 2 spike (delta), B.1.1.529+BA1 (omicron), BA.2 (omicron), B.1.351 (omicron) (BA.2.12.1 (omicron) and BA.5 (omicron) variants. A mean 10-fold boost effect against most variants was observed with a more pronounced effect against more recent variants except for B.617.2 and AY.4 (Figure 13A). In addition, ID50 antibody titers against S, FA-S and RBD were measured to represent antibody binding against those targets. The response in both vaccinated groups remained stable up to 4 weeks post-challenge while no response was observed in the control group. No significant difference could be observed between the two vaccinated groups in term of antibodies targeting either RBD or SΔRBD (Figure 13B). Neutralization assay using infectious virus showed no significant modification of the neutralizing antibodies against D614G but a 10 to 15-fold increase against BA4/5 from 2 weeks pe. Interestingly, unlike the Comirnaty vaccine group, the SΔRBD group presented an initial XBB.1 neutralizing response before challenge. However, by two weeks pe, all three groups showed similar neutralizing responses. In both cases of BA4/5 and XBB.1 the response was maintained in both vaccinated groups, whereas it tended to decline in the control group (Figure 13C). Thus, the general polyvalence of the neutralizing responses has been confirmed with two different assays without any marked difference between the Comirnaty and the SΔRBD group up to four weeks pe.

### Conclusions

In order to focus the immune response away from RBD which accounts for 44% of S mutations (15 in total) in the original Omicron variant B.1.1.529, the inventors developed a SΔRBD antigen coupled to lipid vesicles as a nanoparticle vaccine candidate. They show that the structure of S without RBD folds into a conformation that is anterior to the post-fusion conformation (especially in the prefusion S conformation), which was stabilized by crosslinking, revealing one major site that crosslinks inter S2 subunits as reported for complete recombinant SARS-CoV-2 S glycoprotein S trimer of the invention.

Since a large number of the population has been vaccinated at least twice {57}, the inventors evaluated if a single booster dose with SΔRBD LVs enhances S2-specific antibodies and analyzed their contribution to neutralization after two mRNA vaccinations with Comirnaty. Intermuscular delivery of the vaccine induced robust immunogenicity, mobilizing both humoral and cellular compartments. Throughout the study, no adverse events were recorded in the animals, supporting the safety of the vaccine. Total antibody titers were similar in both the Comirnaty and the SΔRBD groups, indicating that the second boost with SΔRBD LVs is immunogenic and boosts non-RBD antibodies targeting S2 and likely NTD to similar overall S-antibody titers in both groups. SΔRBD LVs likely increased NTD-specific antibodies in addition to S2 antibodies. NTD is highly glycosylated and the early Omicron strain BA1.1.529 contains 8 mutations compared to 15 in RBD and two major neutralizing sites including antigenic supersite 1 that is recognized by most NTD-specific neutralizing antibodies {58; 59; 60, 61}.

They inventors show that serum neutralization activities in the Comirnaty and the SΔRBD groups were similar, suggesting indirectly that non-RBD antibodies generated by the SΔRBD LV boost contribute to neutralization of wild type and omicron strains IN2, BA.1 and BA.4/5. In line with neutralization results, reduced viral burden was measured in nasal, tracheal and lung fluids after challenge with omicron variant XBB3, a subvariant of BA.2.75, which is also closely related to BA4/5. Notably, XBB subvariants have been reported to efficiently evade bivalent mRNA booster as well as previous mRNA vaccination (Wuhan strain) followed by a breakthrough Omicron infection {20; 33; 62; 32}. Furthermore, the SΔRBD group revealed no detectable gRNA and sgRNA in tracheal fluids indicating that the SΔRBD LV boost protects from lung infection. We suggest that this is an important difference between the Comirnaty and SΔRBD groups, because lung infection is a hallmark of Covid-19. Although most omicron variants show reduced lung infection associated with virus attenuation, others can revert, such as BA2.86 revealing efficient lung cell infection {63; 64}. Vaccine-induced protection from lung infection may be due to the liposomal vaccine formulation that can induce mucosal immune responses upon systemic immunization {65}. In agreement with this hypothesis, the inventors detected modest IgG and IgA titers in tracheal fluids upon immunization with LVs harboring full-length S, which provided sterilizing immunity upon virus challenge {40}. Because immunity was not sterilizing in the present study, IgA abundance and specificity were not investigated. Current licensed SARS-CoV-2 vaccines do not induce mucosal immunity and thus are poorly efficient at preventing infection or airway shedding of the virus, although different approaches suggest feasibility of mucosal protection in preclinical models with life attenuated vaccines {66; 67, 68} and exosome-based RBD-decorated antigens {69}.

In summary, the inventors show that SΔRBD LVs are highly immunogenic and allow to redirect the immune response away from immunodominant RBD. The results hint at a skewed repertoire towards non-RBD epitopes which could lead, in combination with existing vaccines, to a lower threshold of immune escape of the SARS-CoV-2 virus. Furthermore, a major drawback of current licensed vaccines is that they are subject to cellular and humoral immunity waning over time {70; 71}. Therefore mRNA primed and boosted with a heterologous vaccines, such as protein-based ones might have several advantages, such as broader and/or more robust and longer-lasting immune responses {72; 73; 74}.

### References

1. Yan et al. (2020). Science 367, 1444-1448
2. Lan et al. (2020). Nature 581, 215-220
3. Guan, X., Yang, Y. & Du, L. Advances in SARS-CoV-2 receptor-binding domain-based COVID-19 vaccines. Expert Rev Vaccines 22, 422-439 (2023)
4. Piccoli, L. et al. Mapping Neutralizing and Immunodominant Sites on the SARS-CoV-2 Spike Receptor-Binding Domain by Structure-Guided High-Resolution Serology. Cell 183, 1024-1042 e1021 (2020)
5. Premkumar, L. et al. The receptor binding domain of the viral spike protein is an immunodominant and highly specific target of antibodies in SARS-CoV-2 patients. Sci Immunol 5, eabc8413 (2020)
6. Stamatatos, L. et al. mRNA vaccination boosts cross-variant neutralizing antibodies elicited by SARS-CoV-2 infection. Science 372, 1413-1418 (2021)
7. Amanat, F. et al. SARS-CoV-2 mRNA vaccination induces functionally diverse antibodies to NTD, RBD, and S2. Cell 184, 3936-3948 e3910 (2021)
8. Deng et al. (2020). Science 369, 818-823
9. Liang et al. (2021). Nat Commun 12, 1346
10. Markov, P. V. et al. The evolution of SARS-CoV-2. Nat Rev Microbiol 21, 361-379 (2023)
11. Chen, Q., Zhang, J., Wang, P. & Zhang, Z. The mechanisms of immune response and evasion by the main SARS-CoV-2 variants. iScience 25, 105044 (2022)
12. Willett, B. J. et al. SARS-CoV-2 Omicron is an immune escape variant with an altered cell entry pathway. Nat Microbiol 7, 1161-1179 (2022)
13. Wang, Q. et al. Alarming antibody evasion properties of rising SARS-CoV-2 BQ and XBB subvariants. Cell 186, 279-286 e278 (2023)
14. Cox, M. et al. SARS-CoV-2 variant evasion of monoclonal antibodies based on in vitro studies. Nat Rev Microbiol 21, 112-124 (2023)
15. Zhou, J. et al. Omicron breakthrough infections in vaccinated or previously infected hamsters. Proc Natl Acad Sci U S A 120, e2308655120 (2023)
16. Zhang, J. et al. Structural and functional characteristics of the SARS-CoV-2 Omicron subvariant BA.2 spike protein. Nat Struct Mol Biol 30, 980-990 (2023)
17. Cao, Y. et al. Omicron escapes the majority of existing SARS-CoV-2 neutralizing antibodies. Nature 602, 657-663 (2022)
18. Planas, D. et al. Considerable escape of SARS-CoV-2 Omicron to antibody neutralization. Nature 602, 671-675 (2022)
19. Dejnirattisai, W. et al. SARS-CoV-2 Omicron-B.1.1.529 leads to widespread escape from neutralizing antibody responses. Cell 185, 467-484 e415 (2022)
20. Wang, Q. et al. Alarming antibody evasion properties of rising SARS-CoV-2 BQ and XBB subvariants. Cell 186, 279-286 e278 (2023)
21. Cao, Y. et al. Imprinted SARS-CoV-2 humoral immunity induces convergent Omicron RBD evolution. Nature 614, 521-529 (2023)
22. Tsang, N. N. Y., So, H. C., Cowling, B. J., Leung, G. M. & Ip, D. K. M. Effectiveness of BNT162b2 and CoronaVac COVID-19 vaccination against asymptomatic and symptomatic infection of SARS-CoV-2 omicron BA.2 in Hong Kong: a prospective cohort study. Lancet Infect Dis 23, 421-434 (2023)
23. Altarawneh, H. N. et al. Effects of Previous Infection and Vaccination on Symptomatic Omicron Infections. N Engl J Med 387, 21-34 (2022)
24. Tuekprakhon, A. et al. Antibody escape of SARS-CoV-2 Omicron BA.4 and BA.5 from vaccine and BA.1 serum. Cell 185, 2422-2433 e2413 (2022)
25. Braeye, T. et al. Vaccine effectiveness against transmission of alpha, delta and omicron SARS-COV-2-infection, Belgian contact tracing, 2021-2022. Vaccine 41, 3292-3300 (2023)
26. Moss, P. The T cell immune response against SARS-CoV-2. Nat Immunol 23, 186-193 (2022)
27. Collie, S., Champion, J., Moultrie, H., Bekker, L. G. & Gray, G. Effectiveness of BNT162b2 Vaccine against Omicron Variant in South Africa. N Engl J Med 386, 494-496 (2022)
28. Lau, J. J. et al. Real-world COVID-19 vaccine effectiveness against the Omicron BA.2 variant in a SARS-CoV-2 infection-naive population. Nat Med 29, 348-357 (2023)
29. Garcia-Beltran, W. F. et al. mRNA-based COVID-19 vaccine boosters induce neutralizing immunity against SARS-CoV-2 Omicron variant. Cell 185, 457-466 e454 (2022)
30. Cele, S. et al. Omicron extensively but incompletely escapes Pfizer BNT162b2 neutralization. Nature 602, 654-656 (2022)
31. Chalkias, S. et al. A Bivalent Omicron-Containing Booster Vaccine against Covid-19. N Engl J Med 387, 1279-1291 (2022)
32. Davis-Gardner, M. E. et al. Neutralization against BA.2.75.2, BQ.1.1, and XBB from mRNA Bivalent Booster. N Engl J Med 388, 183-185 (2023)
33. Kurhade, C. et al. Low neutralization of SARS-CoV-2 Omicron BA.2.75.2, BQ.1.1 and XBB.1 by parental mRNA vaccine or a BA.5 bivalent booster. Nat Med 29, 344-347 (2023)
34. Bianchini, F. et al. Human neutralizing antibodies to cold linear epitopes and subdomain 1 of the SARS-CoV-2 spike glycoprotein. Sci Immunol 8, eade0958 (2023)
35. Caniels, T. G. et al. Emerging SARS-CoV-2 variants of concern evade humoral immune responses from infection and vaccination. Sci Adv 7, eabj5365 (2021)
36. Schmidt, F. et al. Measuring SARS-CoV-2 neutralizing antibody activity using pseudotyped and chimeric viruses. J Exp Med 217, e20201181 (2020)
37. Lescure, F. X. et al. Clinical and virological data of the first cases of COVID-19 in Europe: a case series. Lancet Infect Dis 20, 697-706 (2020)
38. Wrapp, D. et al. Cryo-EM structure of the 2019-nCoV spike in the prefusion conformation. Science 367, 1260-1263 (2020)
39. Hsieh, C. L. et al. Structure-based design of prefusion-stabilized SARS-CoV-2 spikes. Science 369, 1501-1505 (2020)
40. Sulbaran, G. et al. Immunization with synthetic SARS-CoV-2 S glycoprotein virus-like particles protects macaques from infection. Cell Rep Med 3, 100528 (2022)
41. Scheres, S. H. RELION: implementation of a Bayesian approach to cryo-EM structure determination. J Struct Biol 180, 519-530 (2012)
42. Kandiah, E. et al. CM01: a facility for cryo-electron microscopy at the European Synchrotron. Acta Crystallogr D Struct Biol 75, 528-535 (2019)
43. Zheng, S. Q. et al. MotionCor2: anisotropic correction of beam-induced motion for improved cryo-electron microscopy. Nat Methods 14, 331-332 (2017)
44. Zhang, K. Gctf: Real-time CTF determination and correction. J Struct Biol 193, 1-12 (2016)
45. Kimanius, D., Dong, L., Sharov, G., Nakane, T. & Scheres, S. H. W. New tools for automated cryo-EM single-particle analysis in RELION-4.0. Biochem J 478, 4169-4185 (2021)
46. Bepler, T. et al. Positive-unlabeled convolutional neural networks for particle picking in cryo-electron micrographs. Nat Methods 16, 1153-1160 (2019)
47. Cardone, G., Heymann, J. B. & Steven, A. C. One number does not fit all: mapping local variations in resolution in cryo-EM reconstructions. J Struct Biol 184, 226-236 (2013)
48. Sanchez-Garcia, R. et al. DeepEMhancer: a deep learning solution for cryo-EM volume post-processing. Commun Biol 4, 874 (2021)
49. Pettersen, E. F. et al. UCSF Chimera--a visualization system for exploratory research and analysis. J Comput Chem 25, 1605-1612 (2004)
50. Wang, R. Y. et al. Automated structure refinement of macromolecular assemblies from cryo-EM maps using Rosetta. Elife 5, e17219 (2016)
51. Adams, P. D. et al. PHENIX: a comprehensive Python-based system for macromolecular structure solution. Acta Crystallogr D Biol Crystallogr 66, 213-221 (2010)
52. Emsley, P., Lohkamp, B., Scott, W. G. & Cowtan, K. Features and development of Coot. Acta Crystallogr D Biol Crystallogr 66, 486-501 (2010)
53. Williams, C. J. et al. MolProbity: More and better reference data for improved all-atom structure validation. Protein Sci 27, 293-315 (2018)
54. Goddard, T. D. et al. UCSF ChimeraX: Meeting modern challenges in visualization and analysis. Protein Sci 27, 14-25 (2018)
55. Corman, V. M. et al. Detection of 2019 novel coronavirus (2019-nCoV) by real-time RT-PCR. Euro Surveill 25, 2000045 (2020)
56. Wolfel, R. et al. Virological assessment of hospitalized patients with COVID-2019. Nature 581, 465-469 (2020)
57. cdc.gov & ecdc.europe.eu. (https://covid.cdc.gov/covid-data-tracker/#vaccination-archive-landing and https://www.ecdc.europa.eu/en/publications-data/covid-19-vaccine-tracker. (2024).
58. McCallum, M. et al. N-terminal domain antigenic mapping reveals a site of vulnerability for SARS-CoV-2. Cell 184, 2332-2347 (2021)
59. Cerutti, G. et al. Potent SARS-CoV-2 neutralizing antibodies directed against spike N-terminal domain target a single supersite. Cell Host Microbe 29, 819-833 e817 (2021)
60. Cerutti, G. et al. Neutralizing antibody 5-7 defines a distinct site of vulnerability in SARS-CoV-2 spike N-terminal domain. Cell Rep 37, 109928 (2021)
61. Voss, W. N. et al. Prevalent, protective, and convergent IgG recognition of SARS-CoV-2 non-RBD spike epitopes. Science 372, 1108-1112 (2021)
62. Uraki, R. et al. Humoral immune evasion of the omicron subvariants BQ.1.1 and XBB. Lancet Infect Dis 23, 30-32 (2023)
63. Qu, P. et al. Immune evasion, infectivity, and fusogenicity of SARS-CoV-2 BA.2.86 and FLip variants. Cell (2024)
64. Zhang, L. et al. SARS-CoV-2 BA.2.86 enters lung cells and evades neutralizing antibodies with high efficiency. Cell (2024)
65. Bernasconi, V., Norling, K., Bally, M., Hook, F. & Lycke, N. Y. Mucosal Vaccine Development Based on Liposome Technology. J Immunol Res 2016, 5482087 (2016)
66. Hassan, A. O. et al. A Single-Dose Intranasal ChAd Vaccine Protects Upper and Lower Respiratory Tracts against SARS-CoV-2. Cell 183, 169-184 e113 (2020)
67. Alu, A. et al. Intranasal COVID-19 vaccines: From bench to bed. EBioMedicine 76, 103841 (2022)
68. Nouailles, G. et al. Live-attenuated vaccine sCPD9 elicits superior mucosal and systemic immunity to SARS-CoV-2 variants in hamsters. Nat Microbiol 8, 860-874 (2023)
69. Wang, Z. et al. Exosomes decorated with a recombinant SARS-CoV-2 receptor-binding domain as an inhalable COVID-19 vaccine. Nat Biomed Eng 6, 791-805 (2022)
70. Evans, J. P. et al. Neutralizing antibody responses elicited by SARS-CoV-2 mRNA vaccination wane over time and are boosted by breakthrough infection. Sci Transl Med 14, eabn8057 (2022)
71. Ozbay Kurt, F. G. et al. Booster dose of mRNA vaccine augments waning T cell and antibody responses against SARS-CoV-2. Front Immunol 13, 1012526 (2022)
72. Costa Clemens, S. A. et al. Heterologous versus homologous COVID-19 booster vaccination in previous recipients of two doses of CoronaVac COVID-19 vaccine in Brazil (RHH-001): a phase 4, non-inferiority, single blind, randomised study. Lancet 399, 521-529 (2022)
73. Takano, T. et al. Heterologous SARS-CoV-2 spike protein booster elicits durable and broad antibody responses against the receptor-binding domain. Nat Commun 14, 1451 (2023)
74. Zuo, F. et al. Heterologous immunization with inactivated vaccine followed by mRNA-booster elicits strong immunity against SARS-CoV-2 Omicron variant. Nat Commun 13, 2670 (2022)

## Claims

1. Recombinant SARS-CoV-2 S glycoprotein trimer comprising three protomers each comprising a subunit S1 (S1) which may be identical or different in each protomer and a subunit S2 (S2) which may be identical or different in each protomer, **characterised in that** at least one of the protomer is a recombinant protomer comprising a subunit S1 devoid of receptor-binding domain (RBD),
wherein said recombinant SARS-CoV-2 S glycoprotein trimer has a conformation that is anterior to the post-fusion conformation.

2. Recombinant SARS-CoV-2 S glycoprotein trimer according to claim 1, wherein said recombinant protomer comprises a non-antigenic linker linking N-terminal domain (NTD) and subunit S2, said non-antigenic linker preferably being an amino acid motif of sequence consisting of at least three amino acids, more preferably the amino acid motif of sequence consisting of amino acid sequence SEQ ID NO: 12.

3. Recombinant SARS-CoV-2 S glycoprotein trimer according to claim 1 or 2, wherein said recombinant protomer comprises a C-terminal domain (CTD) and a terminal linker linking CTD end, said terminal linker being a poly-histidine tag selected from the group consisting of:
- non-covalent linker with linkage His-tag interaction with DGS-NTA lipids, such as one of the amino acid sequences chosen among amino acid sequences SEQ ID NO: 13, SEQ ID NO: 14 and SEQ ID NO: 15,
- covalent linker having an exposed C-terminal cysteine residue for a covalent linkage via with lipids containing a SH group, such as a linker consisting of the amino acid sequence SEQ ID NO: 5, lipids containing a bromoacetic acid cholesterol or lipids containing a cholesterol-PEG12-maleimide,
- linker with cholesterol modification of the C-terminus cysteine or any other exposed cysteine residue for spontaneous membrane anchoring.

4. Recombinant SARS-CoV-2 S glycoprotein trimer according to one of claims 1 to 3, wherein said recombinant protomer comprises a cleavage site situated between subunit S1 and subunit S2, said cleavage site being preferably a furin or furin-like cleavage site.

5. Recombinant SARS-CoV-2 S glycoprotein trimer according to one of claims 1 to 4, wherein the subunits S2 of at least two of the three protomers are cross-linked to each other, preferably the subunits S2 of the three protomers being cross-linked to each other.

6. Recombinant SARS-CoV-2 S glycoprotein trimer according to claim 5, wherein said subunits S2 comprise each at least one engineered cystein mutant forming inter-protomers disulfide bonds, preferably said at least one engineered cystein being formed by at least one of the further engineered modifications made in the amino acid sequence of SEQ ID NO: 1:
- asparagine residue at position 786 is replaced by cysteine residue and threonine residue at position 355 is replaced by cysteine residue,
- asparagine residue at position 777 is replaced by cysteine residue and glutamine residue at position 563 is replaced by cysteine residue.

7. Recombinant SARS-CoV-2 S glycoprotein trimer according to one of claims 1 to 6, wherein at least one epitope of N-terminal domain (NTD) of said recombinant protomer is linked to a glycan, preferably at least one of the further engineered modifications is made in the amino acid sequence of SEQ ID NO: 1:
- phenylalanine residue at position 135 is replaced by asparagine residue and asparagine 137 is replaced by serine,
- arginine residue at position 158 is replaced by asparagine residue and tyrosine residue at position 160 is replaced by threonine residue,
- glycine residue at position 89 is replaced by serine residue,
- lysine residue at position 187 is replaced by serine residue,
- arginine residue at position 190 is replaced by serine residue,
- valine residue at position 213 is replaced by serine residue,
- arginine residue at position 791 is replaced by asparagine residue,
- aspartic acid residue at position 793 is replaced by serine residue.

8. Recombinant SARS-CoV-2 S glycoprotein trimer according to one of claims 1 to 7, wherein at least one epitope of subunit S2 is unliked to the glycan that initially hid said epitope, preferably at least one arginine residue chosen among arginine residues: N882, N906, N942, N966, N981 and N1002 of the amino acid sequence of SEQ ID NO: 1, is substituted by a glutamine residue.

9. Recombinant SARS-CoV-2 S glycoprotein trimer according to one of claims 1 to 8, which has a pre-fusion conformation.

10. Recombinant SARS-CoV-2 S glycoprotein trimer according to one of claims 1 to 9, wherein said recombinant protomer has an amino acid sequence selected from the group consisting of the sequences having at least 80% amino acid identity with the amino acid sequence of SEQ ID NO: 1.

11. Recombinant SARS-CoV-2 S glycoprotein trimer according to one of claims 1 to 10, wherein the three protomers are devoid of receptor-binding domain (RBD), the three protomers having an amino acid sequence selected from the group consisting of the sequences having at least 80% amino acid identity with the amino acid sequence of SEQ ID NO: 1.

12. Recombinant SARS-CoV-2 S glycoprotein trimer according to one of claims 1 to 11, which is a homomeric trimer.

13. Recombinant SARS-CoV-2 S glycoprotein trimer according to one of claims 1 to 12, wherein said recombinant SARS-CoV-2 S glycoprotein trimer is formulated as mRNA, preferably, said recombinant SARS-CoV-2 S glycoprotein trimer being formulated as mRNA-lipid nanoparticles (LNP).

14. Method of producing a recombinant SARS-CoV-2 S glycoprotein trimer according to one of claims 1 to 13, comprising the following steps:
- expressing nucleic acid molecule(s) encoding said recombinant protomer(s) in a host cell to produce said recombinant SARS-CoV-2 S glycoprotein trimer, and
- purifying said recombinant SARS-CoV-2 S glycoprotein trimer.

15. Method of producing a recombinant SARS-CoV-2 S glycoprotein trimer according to claim 14, further comprising a step of treating said recombinant SARS-CoV-2 S glycoprotein trimer with formaldehyde.

16. Proteoliposome comprising a lipid vesicle a surface of which is coated by the recombinant SARS-CoV-2 S glycoprotein trimer according to one claims 1 to 13.

17. Proteoliposome according to claim 16, wherein said lipid vesicle comprises 50% to 70% by weight of L-α-phosphatidylcholine, 30% to 45% by weight of cholesterol and 0.1% to 10% by weight of a polyhistidine-tag conjugating lipid, preferably 60% by weight of L-α-phosphatidylcholine, 36% by weight of cholesterol and 4% by weight of a polyhistidine-tag conjugating lipid.

18. Method of preparing a proteoliposome according to claim 16 or 17, comprising the step of incubating a recombinant SARS-CoV-2 S glycoprotein trimer according to any of claims 1 to 13 with a lipid vesicle.

19. Vaccine comprising a recombinant SARS-CoV-2 S glycoprotein trimer according to one of claims 1 to 13 or a proteoliposome according to claim 16 or 17 and a pharmaceutically acceptable carrier and/or adjuvant.

20. Vaccine according to claim 19, in a unit dose comprising 10 to 100 µg of said proteoliposome.

21. Vaccine according to claim 19 or 20 for use for the preventive or curative treatment of a coronavirus infection, in particular a SARS-CoV-2 infection.
